# FASCICULE DE BREVET EUROPEEN

(11) **EP 1 056 435 B1**
(45) Date de publication et mention de la délivrance du brevet: **28.06.2006**
(21) Numéro de dépôt: 99961142.9
(22) Date de dépôt: 22.12.1999
(51) Int. Cl.: A61K 8/49, A61K 8/41, A61Q 5/10

(54) **PROCEDE DE TEINTURE DES CHEVEUX UTILISANT UNE AMINE CATIONIQUE HETEROCYCLIQUE ET UN ALDEHYDE OU CETONE OU QUINONE OU DERIVES DE LA DI-IMINO-ISOINDOLINE OU DE LA 3-AMINO-ISOINDOLINE**
VERFAHREN ZUR HAARFÄRBUNG UNTER VERWENDUNG VON EINEM KATIONISCHEN HETEROCYKLICHEN AMIN UND EINEM ALDEHYD ODER EINEM KETON, EINER CHINOVERBINDUNG ODER DERIVATEN VON DIIMINO-ISOINDOLIN ODER 3-AMINO-ISOINDOLON
DYEING METHOD USING AN HETEROCYCLIC CATIONIC AMINE AND A ALDEHYDE OR KETONE OR QUINONE OR DI-IMINO-ISOINDOLINE OR 3-AMINO-ISOINDOLONE DERIVATIVES

(30) Priorité: 23.12.1998 FR 9816377
(43) Date de publication de la demande: 06.12.2000
(73) Titulaire: L'ORÉAL, 75008 Paris (FR)
(72) Inventeur: LAGRANGE, Alain, F-77700 Coupvray (FR); ANDREAN, Hervé, F-75014 Paris (FR)
(74) Mandataire: Casalonga, Axel
(86) Numéro de dépôt international: PCT/FR1999/003248
(87) Numéro de publication internationale: WO 2000/038641

(56) Documents cités:
- EP-A- 0 502 783
- EP-A- 0 847 749
- DE-A- 4 314 317
- DE-A- 4 409 143
- GB-A- 2 181 750

## Description

La présente invention est relative à l'utilisation pour la teinture des fibres kératiniques d'au moins une amine cationique hétérocyclique telle que définie dans la revendication 1 et d'au moins un composé choisi parmi un aldéhyde, une cétone, une quinone et un dérivé de la di-imino-isoindoline ou de la 3-amino-isoindolonc, aux compositions tinctoriales comprenant l'association de ces composés, aux procédés de teinture mettant en oeuvre lesdits composés et à un dispositif à plusieurs compartiments renfermant ces composés.

Pour la teinture des fibres kératiniques, et en particulier des fibres kératiniques humaines telles que les cheveux, il est connu d'utiliser des colorants directs ou substances colorées qui confèrent à la fibre une coloration temporaire ou semi-permanente, de faible puissance tinctoriale et qui s'élimine généralement aux lavages. Les gammes des nuances obtenues par ces procédés directs sont en général réduites. Il est également connu d'utiliser des colorants d'oxydation (bases d'oxydation et coupleurs) qui sont des composés initialement incolores ou faiblement colorés, engendrant sous l'action d'un oxydant, des composés colorés par un processus de condensation oxydative. Les colorations d'oxydation sont, comparativement aux colorations directes, permanentes, puissantes, et résistantes aux agents extérieurs (lumière, intempéries, lavages, transpiration et frottements). Néanmoins, l'utilisation de l'agent oxydant peut altérer les fibres kératiniques et rend les procédés de mise en oeuvre des teintures oxydatives relativement complexes.

La demanderesse vient de découvrir un nouveau procédé de teinture, ne mettant pas oeuvre un processus de développement des colorants par voie oxydative, permettant d'obtenir une large gamme de nuances.

Les composés utilisés par la demanderesse sont de petites molécules qui peuvent facilement pénétrer dans la kératine. La demanderesse a constaté, de façon surprenante, que ces composés peuvent ensuite se condenser en chromophores ou colorants, molécules plus volumineuses qui restent piégées au sein de la kératine.

La demanderesse a ainsi constaté que les colorations obtenues sont résistantes aux shampooings et à la transpiration, stables à la lumière, aux intempéries et aux agents chimiques. Ces colorations sont particulièrement bien résistantes aux shampooings. En quelque sorte, la demanderesse a découvert un nouveau procédé de teinture présentant les avantages de la teinture dite d'oxydation sans en présenter les inconvénients, aucun agent oxydant n'étant utilisé.

La présente invention a donc pour objet l'utilisation pour la teinture des fibres kératiniques d'une amine cationique hétérocyclique et d'un composé choisi parmi un aldéhyde, une cétone, une quinone et un dérivé de la di-imino-isoindoline ou de la 3-amino-isoindolone.

Un autre objet de l'invention est relatif aux compositions de teintures comprenant ces composés.

La présente invention a aussi pour objet un procédé de teinture des fibres kératiniques consistant à appliquer sur les fibres une amine cationique hétérocyclique telle que définie dans la revendication 1 et un composé choisi parmi un aldéhyde, une cétone, une quinone et un dérivé de la diiminoisoindoline ou de la 3-amino-isoindolone, soit simultanément, sous forme d'un mélange extemporané, soit de façon successive.

Un autre objet de l'invention consiste aussi en un agent de teinture pour la mise en oeuvre du procédé de l'invention.

D'autres objets de l'invention apparaîtront à la lumière de la description et des exemples qui suivent.

L'objet principal de la présente invention est donc l'utilisation pour la teinture des fibres kératiniques, en particulier des fibres kératiniques humaines telles que les cheveux humains, d'au moins une amine cationique hétérocyclique telle que définie dans la revendication 1 et d'au moins un composé choisi parmi un aldéhyde, une cétone, une quinone, et un dérivé de la di-imino-isoindoline ou de la 3-amino-isoindolone permettant d'obtenir, par réaction sans agent oxydant, une coloration desdites fibres kératiniques.

On entend par amine cationique hétérocyclique au sens de l'invention, une molécule comprenant au moins une fonction amine, au moins un hétérocycle, de préférence azoté, et au moins un groupement positif, de préférence sur l'hétérocycle.

L'amine cationique hétérocyclique est choisie parmi les composés de formule (I) suivante et leurs sels cosmétiquement acceptables : 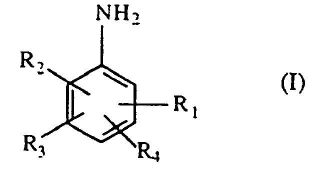 dans laquelle :
- R₁, R₂, R₃, R₄, identiques ou différents, représentent un atome d'hydrogène ; un atome d'halogène, un groupement -NH₂, un groupement -OH, ; un groupement Z ; un groupe -COZ ; un groupe -COOZ ; un radical alkyle carbonyle ; un radical aminoalkyle carbonyle ; un radical N-alkylaminoalkyle carbonyle ; un radical N,N-dialkylaminoalkyle carbonyle ; un radical aminoalkyle carbonylalkyle ; un radical N-alkylaminoalkyle carbonylalkyle ; un radical N,N-dialkylaminoalkyle carbonylalkyle ; un radical carboxy ; un radical alkylcarboxy ; un radical alkylsulfonyle ; un radical aminosulfonyle ; un radical N-alkylaminosulfonyle ; un radical N.N-dialkylaminosulfonyle ; un radical aminosulfonalkyle ; un radical N-alkyl aminosulfonylalkyle ; un radical N,N-dialkyl aminosulfonylalkyle ; un radical carbamyle ; un radical N-alkyl carbamyle ; un radical N,N-dialkylcarbamyle ; un radical carbamylalkyle ; un radical N-alkyl carbamylalkyle ; un radical N,N-dialkyl carbamylalkyle ; un radical alkyle, monohydroxyalkyle, polyhydroxyalkyle, alcoxyalkyle, trifluoroalkyle, un radical cyano ; un groupement ORᵢ, SRᵢ, ORᵢZ ou SRᵢZ ou un groupe amino protégé par un radical alkylcarboxy, trifluoroalkylcarbonyle, aminoalkylcarbonyle, carbonyle, N-alkylaminoalkylcarbonyle, N,N-dialkylaminoalkyl-carbonyle, alkylcarboxy, carbamyle, N-alkylcarbamyle, N,N-dialkylcarbamyle, alkylsulfonyle, aminosulfonyle, N-alkylaminosulfonyle, N,N-dialkylaminosulfonyle, thiocarbamyle, formyle, un groupe -COZ ou un groupe -COOZ ;
- Rᵢ désigne un radical alkyle, monohydroxyalkyle, polyhydroxyalkyle, un groupement Z, un radical alcoxyalkyle ; un radical aryle ; un radical benzyle, un radical carboxyalkyle, un radical alkylcarboxyalkyle, un radical cyanoalkyle, un radical carbamylalkyle, un radical N-alkylcarbamylalkyle ; un radical N,N-dialkylcarbamylakyle ; un radical trifluoroalkyle ; un radical aminosulfonylalkyle ; un radical N-alkylaminosulfonylalkyle ; un radical N,N-dialkylaminosulfonyl-alkyle ; un radical alkylsulfinylalkyle ; un radical alkylsulfonyl-alkyle ; un radical alkylcarbonylalkyle ; un radical aminoalkyle ; un radical aminoalkyle dont l'amine est substituée par un ou deux radicaux identiques ou différents choisis parmi les radicaux alkyle, monohydroxyalkyle ; polyhydroxyalkyle, alkylcarbonyle:, formyle, trifluoroalkylcarbonyle, alkylcarboxy, carbamyle, N-alkylcarbamyle, N,N-dialkylcarbamyle, thiocarbamyle, alkyl-sulfonyle et parmi les groupes Z, -COZ, ou -COOZ ;
Z représentant un groupement de formules (II) ou (III) suivante : 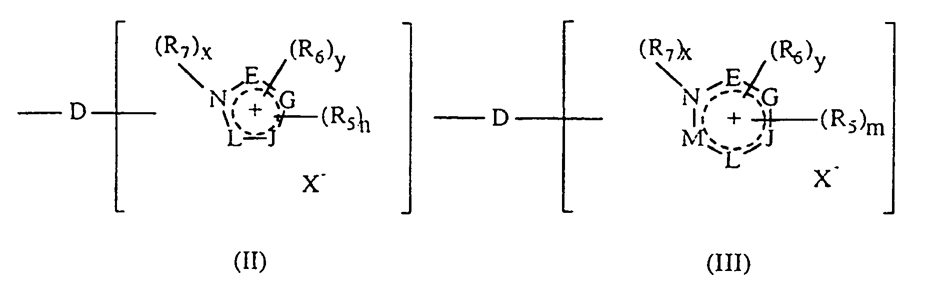 dans lesquelles :
- D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
- les sommets E, G, J, L et M, identiques ou différents, représentent un atome de carbone, d'oxygène, de soufre ou d'azote ;
- n est un nombre entier compris entre 0 et 4 inclusivement ;
- m est un nombre entier compris entre 0 et 5 inclusivement ;
- les radicaux R₅, identiques ou différents, représentent l'une des deux valences d'un bras de liaison D, un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁₋C₆, un radical alkyl(C₁-C₆)thio, un radical amino, un radical amino protégé par un radical alkyl(C₁-C₆)carbonyle, carbamyle ou alkyl(C₁₋C₆)sulfonyle; un groupement NHR" ou NR''R''' dans lesquels R" et R"', identiques ou différents, représentent un radical alkyle en C₁-C₆, un radical monohydroxyalkyle en C₁-C₆ ou un radical polyhydroxyalkyle en C₂-C₆;
- R₆ représente l'une des deux valences d'un bras de liaison D, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁₋C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z;
- R₇ représente l'une des deux valences d'un bras de liaison D, un radical alkyle en C₁-C₆; un radical monohydroxyalkyle en C₁-C₆; un radical polyhydroxyalkylc en C₂-C₆; un radical aryle; un radical benzyle; un radical aminoalkyle en C₁-C₆, un radical aminoalkyle en C₁-C₆ dont l'am.ine est protégée par un radical alkyl(C₁-C₆)carbonyle, carbamyle ou alkyl(C₁-C₆)sulfonyle; un radical carboxyalkyle en C₁₋C₆; un radical cyanoalkyle en C₁-C₆; un radical carbamylalkyle en C₁₋C₆; un radical trifluoroalkyle en C₁-C₆; un radical trialkyl(C₁₋C₆)silanealkyle en C₁-C₆; un radical sulfonamidoalkyle en C₁-C₆; un radical alkyl(C₁-C₆)carhoxyalkyle en C₁-C₆; un radical alkyl(C₁₋C₆)sulfinylalkyle en C₁-C₆; un radical alkyl(C₁-C₆)sulfonylalkyle en C₁-C₆; un radical alkyl(C₁-C₆)cétoalkyle en C₁-C₆; un radical N-alkyl(C₁-C₆)carbamylalkyle en C₁-C₆; un radical N-alkyl(C₁₋C₆)sulfonamidoalkyle en C₁-C₆;
- x et y sont des nombres entiers égaux à 0 ou 1; avec les conditions suivantes:
   - (i) dans les groupements cationiques insaturés de formule (II):
      - lorsque x = 0, le bras de liaison D est rattaché à l'atome d'azote,
      - lorsque x = 1, le bras de liaison D est rattaché à l'un des sommets E, G, J ou L,
   - y ne peut prendre la valeur 1 que:
      1) lorsque les sommets E. G, J et L représentent simultanément un atome de carbone, et que le radical R₆ est porté par l'atome d'azote du cycle insaturé ; ou bien
      2) lorsqu'au moins un des sommets E, G, J et L représente un atome d'azote sur lequel le radical R₆ est fixé;
   - (ii) dans les groupements cationiques insaturés de formule (III):
      - lorsque x = 0, le bras de liaison D est rattaché à l'atome d'azote,
      - lorsque x = 1, le bras de liaison D est rattaché à l'un des sommets E, G, J, Lou M,
   - y ne peut prendre la valeur 1 que lorsqu'au moins un des sommets E, G, J, L et M représente un atome divalent, et que le radical R₆ est porté par l'atome d'azote du cycle insaturé;
- X⁻ représente un anion monovalent ou divalent, et représente de préférence un atome d'halogène tel que le chlore, le brome, le fluor ou l'iode, un hydroxyde, un hydrogènesulfate, ou un alkylsulfate tel que par exemple un méthylsulfate ou un éthylsulfate ; et
- le composé (I) défini ci-dessus présente au moins un groupement Z.

Dans la formule (I) ci-dessus les radicaux alkyle et alcoxy peuvent être linéaires ou ramifiés; les radicaux amino peuvent être éventuellement salifiés par des acides minéraux forts tels que HCl HBr, H₂SO₄, ou des acides organiques tels qu'acétique, lactique, citrique ou succinique.

Parmi les cycles des groupements insaturés Z de formule (II) ci-dessus, on peut notamment citer à titre d'exemple les cycles pyrrolique, imidazolique, pyrazolique, oxcazolique, thiazolique et triazolique.

Parmi les cycles des groupements insaturés Z de formule (III) ci-dessus, on peut notamment citer à titre d'exemple les cycles pyridinique, pyrimidinique, pyrazirtique, oxazinique et triazinique.

Parmi les composés de formule (I), on peut notamment citer :
- le chlorure de 1-[3-(2,4-diamino-phénoxy)-propyl]-3-méthyl-3H-imidazol-1-ium;
- le chlorure de 1-[(3-hydroxy-4-méthyl-phénylcarbamoyl)-méthyl]-3-méthyl-3H-imidazol-1-ium ;
- le chlorure de 3-éthyl-1-[(3-hydroxy-phénylcarbamoyl)-méthyl]-3H-imidazol-1-ium ;
- le chlorure de 1-[(3-hydroxy-2,4-diméthyl-phénylcarbamoyl)-méthyl]-3-méthyl-3H-imidazol-1-ium ;
- le chlorure de 1-[(4-chloro-3-hydroxy-phénylcarbamoyl)-méthyl]-3-méthyl-3H- imidazol-1-ium ;
- le chlorure de 1-[(3-hydroxy-4-méthoxy-phénylcarbamoyl)-méthyl]-3-méthyl-3H-imidazol-1-ium ;
- le chlorure de 1-[(3-hydroxy-4-méthyl-phénylcarbamoyl)-méthyl]-2-méthyl-2H-pyrazol-1-ium ;
- le bromure de 1-[2-(3-hydroxy-4-méthyl-phenylamino)-éthyl]-3-méthyl-3H-imidazol-1-ium ;
- le chlorure de 1-[2-(3-hydroxy-4-méthyl-phénylcirbamoyloxy)-éthyl]-2,3-diméthyl-3H-iniidazol- 1-ium ;
- le dichlorure de 1-{[3-amino-4-(3-(3-méthyl-3H-imidazol-1-ium)-propoxy)-phénylcarbamoyl]-méthyl}-3-methyl-3H-imidazol-1-ium ;
- le dichlorure de 3-(3-triméthylammonium-2-hydroxy-propyl)-1-[(3-hydroxy-4-méthyl-phénylcarbamoyl)-méthyl]-3H-imidarol-1-ium ;
- le chlorure de 1-{[2-(2,4-diamino-phénoxy)-éthylcarbamoyl]-méthyl }-3-méthyl-3H-imidazol-1-ium;
- lechlorure de 1-[(2,4-dihydroxy-phénylcarbamoyl)-méthyl]-3-méthyl-3H-imidazol-1-ium ;
- le dichlorure de N,N-bis-[2-(3-méthyl-3H-imidazol-1-ium)-éthyl]-bcnzene-1,3-diamine ;
- le dichlorure de 1-{3-[4-amino-2-(2-triéthylammonium-acétylamino)-phénoxy]- propyl}-3-méthyl-3H-imidazol-1-ium ;
- le dichlorure de 1-(3-{4-amino-2-[2-(3-méthyl-3H-imidazol-1-ium)-acétylamino]-phénoxy}-propyl)-1,4-diméthyl-pipérazin-1-ium ;
- le chlorure de 1-[2-(2,4-dihydroxy-phényl)-2-oxo-éthyl]-3-méthyl-3H-imidazol-1-ium ;
- le chlorure de 1-[2-(2,4-diamino-phényl)-éthyl]-3-méthyl-3H-imidazol-1-ium ;
- le dichlorure de 1,4-bis-1-{3-[3-(2,4-diamino-phénoxy)-propyl]-3H-imidazol-1- ium}-butane, monohydrate ;
- le chlorure de 1,3-bis-[3-(2,4-diamino-phénoxy)-propyl]-3H-imidazol-1-ium ;
- le chlorure de 3-[3-(2,4-diamino-phénoxy)-propyl]-1-[(3-hydroxy-4-méthyl-phénylcarbamoyl)-méthyl]-3H-imidazol-1-ium ;
- le dichlorure de 1,4-bis-{3-[(3-hydroxy-4-méthyl-phénylcarbamoyl)-méthyl]- 3H-imidazol-1-ium}-butane ;
- le dichlorure de 1,4-bis-{3-[2-(2,4diamino-phényl)-éthyl]-3H-imidazol-1-ium}- butane ;
- le dibromure de 1,4-bis-{3-[2-(3-hydroxy-4-méthyl-phénylamino)-éthyl]-3H-imidazol-1-ium}-butane ;
- le dichlorure de 1,4-bis-{3-[(2,4dihydroxy-phénylcarbamoyl)-méthyl]-3H-imidazol-1-ium}-butane ;
- le chlorure de 3-[3-(2,4-diamino-phénoxy)-propyl]-1-[(2,4dihydroxy-phénylcarbamoyl)-méthyl]-3H-imidazol-1-ium ;
- le monochlorure de 3-[3-(2-amino-phénylamino)-propyl]-1-méthyl-3H-imidazol-1-ium ;
- le monochlorure de 3-[2-(2-amino-phénylamino)-éthyl]-1-méthyl-3H-imidazol-1-ium ;
- le dichlorure de 4-[2-(1-méthyl-3H-imidazol-1-ium)-éthoxy]-N2-[2-(1-méthyl-3H-imidazol-1-ium)-éthyl]-benzène-1,2-diamine ;
- le monochlorure de 3-[2-(2-amino-4-méthyl-phénylamino)-éthyl]-1-éthyl-3H-imidazol-1-ium ;
- lc dichlorure de 3-[3-(2-amino-phénylamino)-propyl]-1-(3-triméthyl-ammonium-2-hydroxy-propyl)-3H-imidazol-1-ium ;
- le monobromure de 3-[3-(2-amino-phénylamino)-propyl]-1-(2-hydroxy-éthyl)-3H-imidazol-1-ium ;
- le monochlorure de 3-{[2-(2-amino-phénylamino)-éthylcarbamoyl]-méthyl}-1-méthyl-3H-imidazol-1-ium ;
- le monochlorure de 1-[2-(2-amino-4-chloro-phénylarnino)-éthyl]-pyridinium ;
- le monochlorure de 3-[2-(2-amino-5-méthoxy-phénylamino)-éthyl]-1-methyl-3H-imidazol-1-ium ;
- le monochlorure de 3-[2-(2-amino-5-méthylsulfanyl-phénylamino)-éthyl]-1-méthyl-3H-imidazol-1-ium ;
- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;
- le chlorure de 3-[3-(4-amino-phénylamino)-propyl]-1-méthyl-3H-imidazol-1-ium ;
- le chlorure de 3-[3-(4-amino-3-méthyl-phénylamino)-propyl]-1-méthyl-3H- imidazol-1-ium ;
- le chlorure de 3-[3-(4-amino-2-méthyl-phénylamino)-propyl]-1-méthyl-3H-imidazol-1-ium ;
- le chlorure de 3-[3-(4-amino-2-fluoro-phénylamino)-propyl]-1-méthyl-3H-imidazol-1-ium, monohydrate ;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-ethyl]-3-méLhyl-3H imidazol-1-ium ;
et leurs sels d'addition avec un acide.

Préférentiellement on utilise comme composés de formule (I), les composés choisis parmi :
- le chlorure de 1-(5-Amino-2-hydroxy-benzyl)-3-méthyl-3H-imidazol-1-iurn ;
- le chlorure de 1-(5-Amino-2-hydroxy-benzyl)-2-méthyl-2H-pyrazol-1-ium ;
- le chlorure de 1-[2-(2,5-Diamino-phényl)-éthyl]-3-méthyl-3H-imidazol-1-ium ;
- le dichlorure de 1,3-bis-1{3{3'[(4"-amino-3"-méthyl-aniline)-N-propyl]}-3H-imidazol-1-iun propane ;
- le dichlorure de 1,3-bis-1{3{3'[(4"-amino-2"-méthyl-aniline)-N-propyl]}-3H-imidazol-1-ium}-propane, monohydrate diéthanol ;
- le dichlorure de 1,3-bis-1{3{3'[(4"-amino-aniline)-N-propyl]}-3H-imidazol-1-ium}-propane, monohydrate éthanol ;
- le dichlorure de 1,3-bis-1{3{3'[(4"-amino-2"-méthyl-aniline)-N-propyl]}-3H-imidazol-1-ium}-2-propanol, monohydrate ;
- le dichlorure de 1,4-bis-1{3[3-(2,5-diamino-phénoxy)-propyl]-3H-imidazol-1-ium}-butane, dihydrate ;
- le mont alorure de 1,3-bis-[3-(2,5-diamino-phénoxy)-propyl]-3H-imidazol-1-ium, monohydrate ;
- le dichlorure de 1,4-bis-1-[3-(5-amino-2-hydroxy-benzyl)-3H-imidazol-lium]- butane, monohydrate ;
- le dibromure de 1,3-bis-{3-(3-[(2-amino-aniline)-N-propyl]}-3H-imidazol-1-ium}-propane ;
- le dichlorure de 1,4-bis-{3-{2-[(2-amino-aniline)-N-éthyl]}-3H-imidazol-1-ium}-butane ;
- le monochlorure de 1-[2-(2-amino-aniline)-éthyl]-3-[3-(2-amino-aniline)-propyl]-3H-imidazol-1-ium ;
et leurs sels d'addition avec un acide.

L'aldéhyde peut correspondre à la formule (IV) suivante: 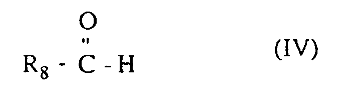 dans laquelle :
R₈ désigne un groupement de formule (IV A) suivante: 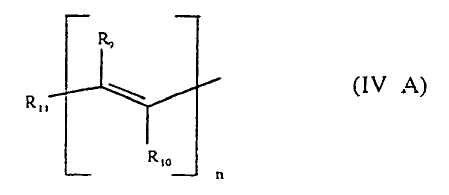
dans laquelle :
R₉ et R₁₀, identiques ou différents, désignent un atome d'hydrogène, un groupement alkyle, mono ou polyhydroxyalkyle, alkylhydroxyalkyle, alcoxy, -CF₃ ou -OCF₃ ,
R₉ et R₁₀ peuvent également former conjointement avec les atomes auxquels ils sont rattachés un cycle aryle ou un hétérocyclique à 5 ou 6 chaînons, lesdits cycles pouvant être substitués ou non;
n désigne un nombre entier de 0 à 3,
R₁₁ désigne les substituants désignés par R₉, un groupement aryle, alkylaryle substitué ou non, un groupe hétérocyclique à 5 ou 6 chaînons substitué ou non,
ou aux sels cosmétiquement acceptables de ces composés.

La cétone peut être choisie parmi les cétones de formules (V) ou (VI) suivantes : 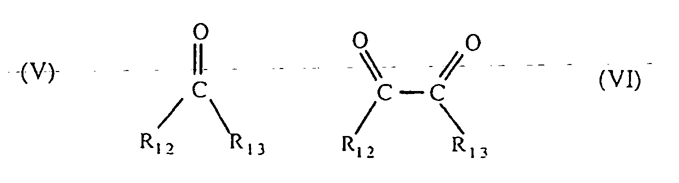 dans lesquelles :
R₁₂ désigne les substituants désignés par R₉,
R₁₃ désigne un groupement alkyle, mono ou polyhydroxyalkyle, alkylhydroxyalkyle, un groupement aryle, alkylaryle, un hétérocyclique à 5 ou 6 chaînons substitué ou non,
R₁₂ et R₁₃ peuvent également former conjointement avec les atomes auxquels ils sont rattachés un cycle aryle à 5 ou 6 chaînons, ou un hétérocyclique comprenant des hétéroatomes tels que N ou S, ledit cycle pouvant lui-même être rattaché à un cycle aryle à 5 ou 6 chaînons ou à un hétérocycle comprenant des hétéroatomes tels que N ou S, lesdits cycles pouvant être substitués ou non,
ou aux sels cosmétiquement acceptables de ces composés.

La quinone peut répondre aux formules (VII) et (VIII) suivantes: 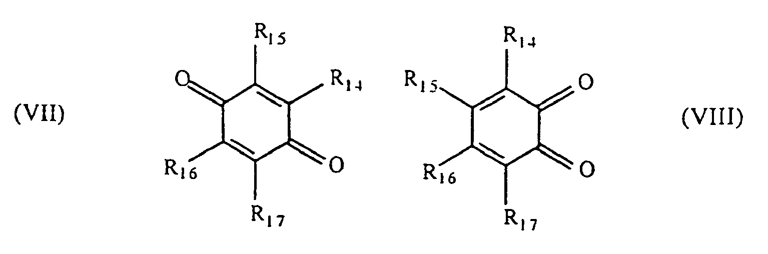 dans lesquelles :
R₁₄ désigne un atome d'hydrogène, d'halogène, un groupement sulfonique ou alcoxy,
R₁₅, R₁₆ et R₁₇, identiques ou différents désignent un atome d'hydrogène, d'halogène, un groupement hydroxy, alkyle, mono ou polyhydroxyalkyle, alkylhydroxyalkyle, alkylsulfonyle, carboxyalkyle, aminoalkyle, alkylaminoalkyle, (di-hydroxy)alkylaminoalkyle, ou alkyle-NR'R" (avec R' et R" désignant alkyle ou pouvant former ensemble avec l'atome d'azote auxquels ils sont rattachés un cycle aryle ou un hétérocycle à 5 ou 6 chaînons), un groupement aryle, un groupe amino pouvant être substitué par un alkyle ou un hydroxyalkyle,
R₁₄ et ₁₅, R₁₅ et R₁₆ ou R₁₆ et R₁₇ peuvent former conjointement avec les atomes auxquels ils sont rattachés un cycle aryle ou un hétérocycle à 5 ou 6 chaînons, substitué ou non;
ou aux sels cosmétiquement acceptables de ces composés.

Les dérivés de la di-imino-isoindoline ou de la 3-amino-isoindolone peuvent être ceux correspondant à la formule (IX) suivante: 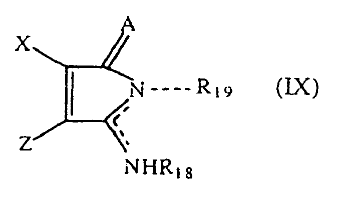 dans laquelle :
R₁₈ et R₁₉, identiques ou différents, désignent un atome d'hydrogène, un groupement alkyle, mono ou polyhydroxyalkyle, alkylhydroxyalkyle, aminoalkyle, alkylaminoalkyle, (dihydroxy)alkylaminoalkyle, ou un groupement alkyle NR'R", avec R' et R" désignant alkyle ou pouvant former conjointement avec l'atome d'azote auxquels ils sont rattachés un cycle aryle ou un hétérocycle à 5 ou 6 chaînons),
A désigne un atome d'oxygène ou NH,
X et Z forment ensemble un cycle aryle ou un hétérocycle à 5 ou 6 chaînons, substitué ou non;
ou aux sels cosmétiquement acceptables de ces composés.

Parmi les composés préférés de formule (IV), on peut notamment citer le benzaldéhyde, les 2,3,4,monohydroxy-benzaldéhydes, les 2,3,4,monométhoxy-benzaldéhydes, les 2,3,4,monométhyl-benzaldéhydes, les (2,3), (2,4), (2.5), (2.6), (3,5)-dihydroxy benzaldéhydes, les (2,3), (2,4), (2,5), (2.6), (3,5)-diméthoxy benzaldéhydes, la vaniline, l'isovaniline, le syringaldéhyde, les ortho, iso, téré-phthaldéhyde, les (2,3), (2,4), (2,5), (2.6), (3,5)-diméthyl-benzaldéhydes, le 4-isopropyl-benzaldéhyde, 4-diméthylamino-benzaldéhyde, 4-diéthylaminol-benzaldéhyde, le pipéronal, les (2,6), (3,5)-diméthyl-4-hydroxy-benzaldéhyde, les 2,3,4-mononitro-benzaldéhydes, le 2-hydroxy-3-méthoxy-benzaldéhyde, le 2-hydroxy-4-méthoxy-benzaldéhyde, le 2-hydroxy-5-méthoxy-benzaldéhyde, le 2-hydroxy-6-méthoxybenzaldéhyde, le 4-méthylthio-benzaldéhyde, les (2,3,4), (2,4,6), (3,4,5), (2,4,5)-trihydroxy-benzaldéhydes, les méthyles 2, 3 et 4-formylbenzoates, les 2,3,4-mono(2-hydroxyethoxy)-benzaldéhydes, le 4-nitro-3-hydroxy-benzaldéhyde, le 3-nitro-4-hydroxy-benzaldéhyde, le 2-nitro-4-hydroxy-benzaldéhyde, le 3-nitro-2-hydroxy-benzaldéhyde, les 2,3,4-monotrifluoro-benzaldéhydes, le 2,3-dihydroxy-4-méthoxy-benzaldéhyde, le 3,4-dihydroxy-5-méthoxy-benzaldéhyde, le 3,5-dihydroxy-4-méthoxy-benzaldéhyde, le 3-méthoxy-2-nitrobenzaldéhyde, le 4-méthoxy-2-nitrobenzaldéhyde, le 2-méthoxy-3-nitrobenzaldéhyde, le 4-méthoxy-3-nitrobenzaldéhyde, les (2,3,4), (2,4,6), (3,4,5), (2,4,5)-triméthoxy-benzaldéhydes, la 5-nitrovaniline, les (2,4), (2,6)-dinitrobenzaldéhydes, le pentaméthyl-benzaldéhyde, le 4-méthylsulfonyl-benzaldéhyde, les acides 2,3,4-monoformylphénoxyacétiques, le 4-diéthylamino-salicylaldéhyde, le 4(3-diméthylaminopropoxy)-benzaldéhyde, le 2,3-dihydrobenzo (b)furan-5-carboxaldéhyde, le 1 et le 2 naphthaldéhyde, le 6 et 5 carboxaldéhyde-1,4-benzodioxane, les 2,4-monuhydroxy-1-naphtaldéhydes, le 1-monohydroxy-2-naphtaldéhyde, le 1(4-formylphényl)-imidazole, le 4-pyrrolidinol-benzaldéhyde, les 2,4 monométhoxy-1-naphthaldéhydes, le 2,3-diméthyl-chroman-6-carboxaldéhyde, le 2,3,6,7-tétrahydro-1H,5H-pyrido(3.2,1-IJ) Quinoline-9-carbaldéhyde, le 4 diméthylamino-1-naphthaldéhyde, le 9-anthraldéhyde, le 3-nitro-4-pyrrolidino-benzaldéhyde, le 3-nitro-4-pipéridino-benzaldéhyde, le 3-nitro-4-morpholino-benzaldéhyde, les pyridines 2,3,4-monocarboxaldéhydes, le 2,6-pyridino-dicarboxaldéhyde, le 5-formyl-6-méthyluracil, le pyridoxale, les quinoléïnes - 2,3,4-monocarboxaldéhydes, le 8-hydroxy-quinoléïne-2-carboxaldéhyde, les 2 et 3-furaldéhydes, les 2 et 3-thiénylcarboxaldéhydes, les 2 et 3-imidazo-carboxaldéhydes, le 2-pyrrolcarboxaldéhyde, le 5-nitro-2-furaldéhyde, le 5-(diméthylamino)-2-furaldéhyde, les 2,5 et 2,3-thiophène-dicarboxaldéhydes, le pyrazol-3-carbaldéhyde, le 5-nitro-2-thiophène-carboxaldéhyde, le 5-nitro-3-thiophénecarboxaldéhyde, l'indole-3-carboxaldéhyde, le N-méthyl-indole-3-carboxaldéhyde, le 2-méthyl-indole-3-carboxaldéhyde, les 4,5,6,7-monométhyl-indole-carboxaldéhyde et l'acide 5-formyl-2-furansulfonique.

Les cétones de formules (V) et (VI) peuvent être choisies parmi la 2,3 indolinedione,la 2,3-butanedione, la 2,3-pentanedione, la (2,3), (3,4)-hexanedione, la 1-phényl-1,2-propanedione, le benzyl, le furil, le 2,2'-pyridil, le nitro-benzyl, l'anisil, le 3.3'-diméthoxybenzyl,le 4,4'-bis(diméthylamino)benzyl, la camphoroquinone, le cyclohexane-1,2-dionc, l'isatine, la N-méthylisatine, la 4,5.6,7-monométhyl-isatine, la (4.5),(4.7),(5.7),(6.7)-diméthyl-isatine, la N-éthyl-isatinc, la N-hydroxyméthyl-isatinc, la 5,6,7 monométhoxy-isatine, la 4,5,6,7 monochloro-isatine, la 4,5,6,7 monobromo-isatine, la N-isopropyl-isatine, la N-butyl-isatine, la N-propyl-isatine, la 5-nitro-isatine, l'acide 5-sulfonique-isatine, la 2,4,5-trihydroxypyrimidine, l'alloxane, la 1,3-diméihyl-hexahydro-2,4,5,6-pyrimidinetetraone, la ninhydrine, la chinisatine, le 1,3-indenedione, l'acide squarique, l'acide eroconique, la 3,4-diméthoxy-3-cyclobutène-1,2-dione, la 3,4-éthoxy-3-cyclobutène-1,2-dione, la 3,4-isopropoxy-3-cyclobutène-1,2-dione, la 3,4-di-N-butoxy-3-cyclobutene-1,2-dione, l'acide rhodizonique, l'oxindole, la N-méthyl-2-indolinone, la N-méthyl-nitro-2-indolinone, le 6-méthoxyoxindole, le 5,6-diméthoxyoxindole et les 5 et 6-monochlorooxindole.

Les quinones préférées de formules (VII) et (VIII) sont, entre autres, la 1,4 naphtoquinone, la spinulosine, l'atromentine, l'aurentioglyocladine, la 2,5-dihydroxy-6-méthylbenzoquinone, la 2-hydroxy-3-méthyl-6méthoxylbenzoquinone, la 2,5-dihydroxy-3,6-diphénylbenzoquinone, la 2,3-diméthyl-5-hydroxy 6-méthoxybenzoquinone, la 2,5-dihydroxy 6-isopropyl-benzoquinone, la lawsone, la juglone, la fafioline, la naphtazarine, la naphtopurpurine, le lapachol, la plumbagine, la chloroplumbagine, la drosérone, la shikonine, la 2-hydroxy-3-méthyl-1,4-naphtoquinone, la 3,5-dihydroxy-1,4-naphtoquinone, la 2,5-dihydroxy-1,4-naphtoquinone, la 2-méthoxy-5-hydroxy-1,4-naphtoquinone, la 3-mélhoxy-5-hydroxy-1,4-naphtoquinone, la (1,4),(1,2)naphtoquinone, la 4,5-diméthoxy-1,2-benzoquinone, la phenanthrènequinone et l'acide 4-sulfonique(1,2)naphtoquinone.

Les dérivés de formule (IX) sont notamment représentés par la 3-imino-3H-isoindol-ylamine, la 3-imino-4-méthyl-3H-isoindol-1-ylamine, la 3-imino-4-terbutyl-3H-isoindol-1-ylamine, la 3-imino-7-nitro-3H-isoindol-1-ylamine, la 3-amino-1-imino-1H-isoindol-4-ol, la 3-imino-7-isopropoxy-3H-isoindol-1-ylamine, la 3-imino-7-(2,2,2-trifluoroéthoxy)-3H-isoindol-1-ylamine, la 3-imino-7-éthoxy-3H-isoindol-1-ylamine, la 3-imino-7-butoxy-3H-isoindol-1-ylamine, l'acide 3-amino-1-imino-1H-isoindole-4-sulfonique, la 3-imino-7-chloro-3H-isoindol-1-ylamine, la 3-imino-5-niéthyl-3H-isoindol-1-yiaminc, la 3-imino-5-éthyl-3H-isoindol-1-ylamine, la 3-imino-5-terbuij,1-3H-isoindol-1-j,lamine, la 3-imino-5-amino-3H-isoindol-1-ylamine, la N-(1-amino-3-imino-3H-isoindol-5-yl)-acétamide, la 3-imino-5-nitro-3H-isoindol-1-ylamine, la 3-iminc>-5-fluoro-3H-isoindol-1-ylarnine, la 3-imino-5-chloro-3H-isoindol-1-ylamine, la 3-imino-5-méthylsulfanyl-3H-isoindol-1-ylamine, la 3-imino-5-méthoxy-3H-isoindol-1-ylamine, la 3-imino-5-éthoxy-3H-isoindol-1-ylamine, la 3-imino-5-propoxy-3H-isoindol-1-ylamine, la 3-imino-5-isopropoxy-3H-isoindol-1-ylamine, la 3-imino-5-butoxy-3H-isoindol-1-ylamine, la 3-imino-5-isobutoxy-3H-isoindol-1-ylamine, la 3-imino-5-terbutoxy-3H-isoindol-1-ylamine, la 3-imino-5-(2,2,2-trifluorométhyl)-3H-isoindol-1-ylamine, la 3-imino-5-(2,2,2-trifluoroéthoxy)-3H-isoindol-1-ylamine, la 3-imino-5-méthanesulfonyl-3H-isoindol-1-ylamine, la 3-imino-5,6-diméthyl-3H-isoindol-1-ylamine, la 3-imino-5,6-diéthyl-3H-isoindol-1-ylamine, la 3-imino-5,6-diméthoxy-3H-isoindol-1-ylamine, la 3-imino-5,6-diéthoxy-3H-isoindol-1-ylamine, la 3-imino-5,6-dibutoxy-3H-isoindol-1-ylamine, la 3-imino-5,6-bis-trifluorométhyl-3H-isoindol-1-ylamine, la 3-imino-5,6-dichloro-3H-isoindol-1-ylamine, la 5,6-bis-éthoxyméthyl-3-imino-3H-isoindol-1-ylamine, la 3-amino-1-imino-1H-isoindol-4,7-diol, la 4,7-dichloro-3-imino-3H-isoindol-1-ylamine, la 4,5,7-trichloro-3-imino-N6,N6-diméthyl-3H-isoindol- 1,6-diamine, la 4,5,6,7-tétrachloro-3-imino-3H-isoindol-1-ylamine, la 4,5,6,7-tétrafluoro-3-imino-3H-isoinciol-1-ylamine, la 3-butyliminu-3H-isoindol-1-ylaminc, la 2-(3-amino-isoindol-1-ylidèneamino)-éthanol, la 3-(3-amino-isoindol-1-ylidèneamino)-3-méthyl-pentane-1.5-diol, la N-(3-amino-isoindol-1-ylidène)-guanidine, la 7-imino-7H-pyrrolo[3,4-b]pyridin-5-ylamine, la 7-imino-7H-pyrrolo[3,4-b]pyrazin-5-ylamine, la 7-imino-2,3-dimémyl-7H-pyrrolo[3,4-b]pyrazin-5-ylamine, la 7-imino-7H-[1,4]dithiino[2,3-c] pyrrol-5-ylamine, la 7-imino-2,3-diméthyl-7H-[1,4]dithiino[2,3-c] pyrrol-5-ylamine, la 7-imino-2,3-dihydro-7H-[1,4]dithiino[2,3-c]pyrrol-5-ylamine, la 7-imino-2-méthyl-2,3-dihydro-7H-[1,4]dithiino [2,3-c]pyrrol-5-ylamine, la 3-amino-isuindol-1-one, la 3-amino-7-méthyl-isoindol-1-one, la 3-amino-7-hydroxyméthyl-isoindol-1-one, la 3-amino-7-chloro-isoindol-1-one, la 3-amino-4-chloro-isoindol-1-one, l'acide 3-amino-1-oxo-1H-isoindole-4-sulfonique, la 3-amino-4-nitro-isoindul-1-one, la 3-amino-6-nitro-isoindol-1-one, la 3-amino-6-méthyl-isoindol-1-one, la 3-amino-6-chloro-isoindol-1-one, la 3-amino-6-bromo-isoindol-1-one, la 3-amino-6-méthylsulfanyl-isoindol-1-one, la 3-amino-6-méthoxy-isoindol-1-one, la 3-amino-5-chluro-isoindol-1-one, la 3-amino-5-fluoro-isoindol-1-one, la 3-amino-5-méthoxy-isoindol-1-one, la 3-amino-5-nitro-isoindol-1-one, l'ester éthylique de l'acide 3-amino-1-oxo-1H-isoindole-5-carboxylique, la 3-amino-5,6-dichloro-isoindol-1-one, la 3-amino-5,6-dibromo-isoindol-1-one, la 3-amino-4,7-dichloro-isoindol-1-one, la 3-amino-4,5,7-trichloro-isoindol-1-one, la 3-amino-4,5,6,7-tétrachloro-isoindol-1-one, la 3-amino-4,5,7-trichloro-6-méthylsulfanyl-isoindol-1-one, la 3-amino-4,5,6,7-tétrabromo-isoindol-1-one, la 3-amino-4,5,6,7-tétrafluoro-isoindol-1-one, la 3-méthylamino-isoindol-1-one, la 3-éthylamino-isoindol-1-une, la 3-propylamino-isoindol-1-one, la 3-diméthylamino-isoindol-1-one, la 7-éthylamino-pyrrolo[3,4-b]pyridin-5-one, la 7 -amino-pyrrolo[3,4-b]pyridin-5-one, la 3-amino-pyrrolo[3,4-c]pyridin-5-one, la 3-amino-6-méthyl-pyrrolo[3,4-c]pyridin-1-one, la 5-amino-pyrrolo[3,4-b]pyridin-7-one, la 7-amino-pyrrolo[3,4-b]pyrazin-5-one, la 7-amino-2-méthyl-pyrrolo[3,4-b]pyrazin-5-one, la 7-amino-2,3-diméthyl-pyrrolo[3,4-b]pyrazin-5-one, la 7-amino-2,3-dihydro-[1,4]dithiino[2,3-c]pyrrol-5-onc, la 3-imino-2-méthyl-2,3-dihydro-isoindol-1-one, la 3-imino-2-éthyl-2,3-dihydro-isoindol-1-one, la 3-imino-2-propyl-2,3-dihydro-isoindol-1-one, la 2-hydroxymétlyl-3-imino-2,3-dihydro-isoindol-1-one, la 2-(2-hydroxyéthyl)-3-imino-2,3-dihydro-isoindol-1-one, l'acide 2-(1-imino-3-oxo-1,3-dihydro-isoindol-2-yl)-éthane sulfonique, l'acide 3-(1-imino-3-oxo-1,3-dihydro-isoindol-2-yl)-propionique, la 2-(3-hydroxypropyl)-3-imino-2,3-dihydro-isoindol-1-one et la 5-imino-6-méthyl-5,6-dihydro-pyrrolo[3,4-b]pyridin-7-one.

Dans le cadre de la présente invention:
Les atomes d'halogène désignent préférentiellement un atome de fluor, de chlore, de bromure ou d'iode.
Les radicaux alkyle, monohydroxyalkyle, polyhydroxyalkyles, alkylhydroxyalkyle, alkylesulfonyle, carboxyalkyle, aminoalkyle, alkylaminoalkyle, dihydroxyaminoalkyle peuvent être linéaires ou ramifiés.
Les groupements alkyle désignent notamment les groupements de 1 à 20 atomes de carbone, comme par exemple, les groupements méthyle, éthyle, propyle, isopropyle, n-propyle, butyle, n-butyle, tert-butyle, pentyle, n-pentyle, isopentyle, n-hexyle, isohexyle, heptyle, octyle, nonyle, decyle, undecyle, dodecyle et pentadecyle. Préférentiellement, les groupements alkyle désignent un groupement de 1 à 6 atomes de carbone ;
ces groupements alkyles peuvent être substitués; par exemple, par un atome d'halogène, un radical cyano ou hydroxy, et peuvent ainsi représenter les radicaux trifluorométhyle, δ-chloropropyle, β-cyanoéthyle ou β-hydroxyéthyle.

Parmi les groupements monohydroxyalkyle, on peut notamment citer les groupements hydroxyméthyle, hydroxyéthyle, hydroxypropyle et hydroxybutyle.

Parmi les radicaux polyhydroxyalkyle, on peut par exemple citer les radicaux dihydroxyéthyle, dihydroxypropyle, trihydroxypropyle et dihydroxybutyle.

Les groupements alcoxy désignent un groupement -O-R, R représentant un groupement alkyle tel que défini ci-dessus.

Les groupements alcényles désignent un radical monovalent correspondant aux carbones éthyléniques, tels que, par exemple, alkyle ou 3,3diméthylallyle.

Les groupements acétyloxy désignent un groupement -O-CO-R, R représentant un groupement alkyle tel que défini ci-dessus.

Parmi les radicaux cycloalkyle, on peut notamment citer le cyclohexyle et le cyclopentyle.

Parmi les radicaux aryle, qui peuvent être mono ou polycycliques, on peut notamment citer les groupements phényle ou naphtyle.

Parmi les hétérocycles et notamment les cycles à 5 ou 6 chaînons, qui peuvent être mono ou polycycliques et contenant un ou plusieurs hétéroatomes, on peut citer les cycles thiophène, pyrrole, imidazole, pyrazole, triazole, thiazole, furane, benzofuranc, benzimidazole, benzothiazole, pyridyle, benzoxazole, quinolyle, quinazoyle, quinoxalyle, pyrrolidine, pipéridine, pipérazine ou morpholine.

Parmi les radicaux alkylaryle, on peut notamment citer le groupement benzyle, phenethyle ou naphthylméthyle.

Les groupements aminoaryle désignent les groupements NH₂₋R, R représentant un radical aryle.

Dans le cadre de la présente invention, les radicaux cycloakyles, aryle et les hétérocycles peuvent être substitués ou polysubstitués par exemple par un halogène, par un alkyle ou monohydroxyalkyle en C₁-C₆, un radical polyhydroxyalkyle en C₂-C₆, un alkoxy en C₁-C₆, un groupe nitro, un groupe hydroxy, un groupe carboxylique, un groupe acétyloxy en C₁-C₄, un groupe carboxamide, un groupe sulfonamide, sulfonique, nitrile, -CF₃ ou -OCF₃, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical alkylcarbonyl en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.

Dans le cadre de la présente invention, les formules (I) à (IX) ne sont pas limitées à celles spécifiquement décrites mais comprennent aussi leurs formes tautomères quand elles existent.

Au sens de la présente invention, les sels cosmétiquement acceptables des composés précités peuvent être des chlorhydrates, des sulfates, des bromhydrates ou des tartrates.

Les compositions de teinture des fibres kératiniques, en particulier des fibres kératiniques humaines telles que les cheveux, conformes à la présente invention sont essentiellement caractérisées par le fait qu'elles comprennent au moins une amine cationique hétérocyclique telle que définie ci-dessus et au moins un composé choisi parmi un aldhéhyde, une cétone, une quinone et un dérivé de la di-imino-isoindoline ou de la 3-amino-isoindolone tel que défini ci-dessus, dans un milieu approprié pour la teinture.

Dans une forme de réalisation préférée de l'invention, le composé choisi parmi un aldéhyde, une cétone, une quinone et un dérivé de la di-imino-isoindoline ou de la 3-amino-isoindolone est choisi parmi la 1,4-diméthylaminobenzaldéhyde et la 4-diméthylaminonaphtaldéhyde.

L'amine cationique hétérocyclique peut être présente dans une concentration allant de 0,01 à 10 %, et préférentiellement entre 0,05 et 5 % en poids par rapport au poids total de la composition.

Le composé choisi parmi un aldéhyde, une cétone, une quinone et un dérivé de la di-imino-isoindoline ou de la 3-amino-isoindolone peut être présent dans une concentration allant de 0,01 à 10 %et préférentiellement de 0,05 à 5 % en poids par rapport au poids total de la composition.

Le milieu approprié pour la teinture est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.

On peut également utiliser des agents épaississants dans une proportion allant d'environ 0,2 à 20%.

Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 2 à 11 et de préférence de 5 à 10, et pouvant être ajusté au moyen d'agents d'alcalinisation ou d'agents d'acidification ou de tampons antérieurement bien connus.

Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 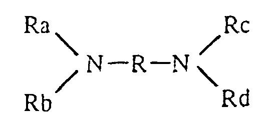 dans laquelle, R est un reste propylène éventucllemcnt substitué par un groupement hydroxyle ou un radical alkyle en C₁-C₄; Ra, Rb, Rc et Rd, simultanément ou indépendamment l'un de l'autre représentent un atome d'hydrogène, un radical alkyle en C₁-C₄ ou hydroxyalkyle en C₁-C₄.

Les agents acidifiants sont classiquement des acides minéraux ou organiques comme par exemple les acides chlorhydriques, tartrique, citrique ct phosphorique.

Parmi les tampons, on peut citer par exemple, le phosphate diacide de potassium/hydroxyde de sodium.

La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Conformément à la présente invention, le procédé de teinture des fibres kératiniques, en particulier des fibres kératiniques humaines telles que les cheveux, est essentiellement caractérisé par le fait que l'on applique sur lesdites fibres un composant (A) constitué d'une composition renfermant dans un milieu approprié pour la teinture, au moins une amine cationique hétérocyclique telle que définie ci-dessus, et un composant (B) constitué d'une composition contenant dans un milieu approprié pour la teinture, au moins un composé choisi parmi un aldehyde, une cétone, une quinone et un dérivé de la di-imino-isoindoline ou de la 3-amino-isoindolone tel que, par exemple, un de ceux définis ci-dessus, de façon à permettre le développement d'une teinture sur lesdites fibres kératiniques.

Dans une forme de réalisation préférée du procédé de l'invention, les composants (A) et (B) sont mélangés juste avant emploi, puis la composition résultante est immédiatement appliquée sur les fibres kératiniques, et laissée agir pendant 1 à 60 minutes et préférentiellement de 1 à 30 minutes ; les fibres kératiniques étant ensuite rincées, lavées au shampooing, rincées à nouveau, puis séchées.

Un autre procédé de la présente invention consiste essentiellement à appliquer sur les fibres kératiniques le composant (A), suivi ou précédée de l'application sur lesdites fibres du composant (B), à laisser agir chaque composant pendant 1 à 60 minutes et préférentiellement de 1 à 30 minutes, à procéder éventuellement au rinçage à l'eau entre chaque application; les fibres kératiniques étant ensuite rincées, lavées au shampooing, rincées à nouveau, puis séchées.

Un objet de l'invention est aussi constitué par un agent de teinture pour les fibres kératiniques, en particulier des cheveux humains, caractérisé par le fait qu'il est constitué par les composants (A) et (B) stockés sous forme séparée, tels que définis ci-dessus.

Les composants (A) et (B) sont destinés, soit à être mélangés tous juste avant emploi, soit à être appliqués de façon successive sur les fibres à traiter.

Selon une forme de réalisation, on peut conditionner les différents composants (A) et (B) dans un dispositif à plusieurs compartiments encore appelé "kit de teinture" comportant tous les composants destinés à être appliqués pour une même teinture sur les fibres kératiniques, en particulier les fibres kératiniques humaines telles que les cheveux, en applications successives avec ou sans prémélange.

De tels dispositifs peuvent comporter un premier compartiment contenant le composant (A) renfermant l'amine cationique hétérocyclique et un second compartiment comportant le composant (B) renfermant le composé choisi parmi un aldéhyde, une cétone, une quinone et un dérivé de la di-imino-isoindoline ou de la 3-amino-isoindolone.

Une autre variante peut également consister à stocker le composant (A) ou le composant (B) dans un milieu solvant anhydre et à prévoir un troisième compartiment contenant un milieu aqueux approprié pour la teinture et cosmétiquement acceptable. Dans ce cas, on mélange tout juste avant l'emploi le contenu du troisième compartiment dans l'un ou l'autre ou les deux compartiments contenant les composants anhydres (A) et (B) ou alors on mélange avant emploi les trois compartiments.

Des exemples concrets illustrant l'invention vont maintenant être donnés.

### EXEMPLE 1

La composition de teinture suivante a été préparée juste avant emploi :
- 1H-indole-2,3-dione (3.10⁻³mole) 0,441 g
- chlorhydrate de chloruer de 3-[3-(4-amino-phénylamino)-propyl]-1-métuyl-3H-imidazolium (3.10⁻³ mole) 1,02 g
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| rouge brun intense | rouge brun intense | rouge brun intense |

Ces colorations sont particulièrement résistantes aux lavages aux shampooings.

### EXEMPLE 2

La composition de teinture suivante a été préparée juste avant emploi :
- 1H-indole-2,3-dione (3.10⁻³mole) 0,441 g
- chlorhydrate de chlorure de 3-[3-(4-amino-phénylamino)-propyl]-1-méthyl-3H-imiciazolium (3.10⁻³ mole) 1,02 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| bois de rose | cuivre rouge clair | cuivre rouge clair |

Ces colorations sont tenaces en particulier vis-à-vis des shampooings.

### EXEMPLE 3

La composition de teinture suivante a été préparée juste avant emploi :
- 1H-indole-2,3-dione (3.10⁻³mole) 0,441 g
- chlorhydrate de chlorure de 1,3-bis-{3-[3-(4-amino-phénylamino)-propyl]-3H-imidazol-1-ium}-propane (3.10⁻³ mole) 2,07 g
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| magenta | magenta très intense | magenta très intense |

Ces colorations sont particulièrement résistantes aux shampooings.

### EXEMPLE 4

La composition de teinture suivante a été préparée juste avant emploi :
- 1H-indole-2,3-dione (3.10⁻³mole) 0,441 g
- chlorhydrate de chlorure de 1,3-bis-{3-[3-(4-amino-phénylamino)-propyl]-3H-imidazol-1-ium}-propane (3.10-³ mole) 2,07 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| bois de rose très intense | cuivre rouge flamboyant | cuivre rouge intense |

Ces colorations sont tenaces en particulier vis-à-vis des shampooings.

### EXEMPLE 5

La composition de teinture suivante a été préparée juste avant emploi :
- 1H-indole-2,3-dione (3.10⁻³mole) 0,441 g
- chlorhydrate de chlorure de 1-[3-(2,5-diamino-phénoxy)-propyl]-3-méthyl-3H-imidazol-1-ium (3.10⁻³ mole) 1,07 g
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| brun rouge léger | brun rouge intense | brun rouge intense |

Ces colorations sont tenaces en particulier vis-à-vis des shampooings.

### EXEMPLE 6

La composition de teinture suivante a été préparée juste avant emploi :
- 1H-indole-2,3-dione (3.10⁻³mole) 0,441 g
- chlorhydrate de chlorure de 1-[3-(2,5-diamino-phénoxy)-propyl]-3-méthyl-3H-imidazol-1-ium (3.10⁻³ mole) 1,07 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permancnlés |
|---|---|---|
| blond rosé | châtaigne clair | brun rouge |

Ces colorations sont tenaces en particulier vis-à-vis des shampooings.

### EXEMPLE 7

La composition de teinture suivante a été préparée juste avant emploi :
- 1-méthyl-1H-indole-2,3-dione (3.10⁻³mole) 0,483 g
- chlorhydrate de chlorure de 3-[3-(4-amino-phénylamino)-propyl]-1-méthyl-3H-imidazolium (3.10⁻³ mole) 1,02 g
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| rouge sombre | rouge sombre à reflet violacé | rouge sombre à reflet violacé |

Ces colorations sont tenaces en particulier vis-à-vis des shampooings.

### EXEMPLE 8

La composition de teinture suivante a été préparée juste avant emploi :
- 1-méthyl-1H-indole-2,3-dione (3.10⁻³mole) 0,483 g
- chlorhydrate de chlorure de 3-[3-(4-amino-phénylamino)-propyl]-1-méthyl-3H-imidazolium (3.10⁻³ mole) 1,02 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| rosé | cuivre rouge | rouge cuivré |

Ces colorations sont tenaces en particulier vis-à-vis des shampooings.

### EXEMPLE 9

La composition de teinture suivante a été préparée juste avant emploi :
- 1-méthyl-1H-indole-2,3-dione (3.10⁻³mole) 0,483 g
- chlorhydrate de chlorure de 1,3bis-{3-[3-(4-amino- 2,07 g phénylamino)-propyl]-3H-imidazol-1-ium}-propane (3.10⁻³ mole)
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| prune | prune intense | prune intense |

Ces colorations sont tenaces en paniculier vis-à-vis des shampooings.

### EXEMPLE 10

La composition de teinture suivante a été préparée juste avant emploi :
- 1-méthyl-1H-indole-2,3-dione (3.10⁻³mole) 0,483 g
- chlorhydrate de chlorure de 1,3bis-{3-[3-(4-amino-phénylamino)-propyl]-3H-imidazol-1-ium}-propane (3.10⁻³ mole) 2,07 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| brun rouge | brun rouge intense | brun rouge intense |

Ces colorations sont tenaces en particulier vis-à-vis des shampooings.

## Revendications

1. Utilisation pour la teinture des fibres kératiniques, en particulier des fibres kératiniques humaines telles que les cheveux humains, d'au moins un composé choisi parmi un aldéhyde, une cétone, une quinone, et un dérivé de la diiminoisoindoline ou de la 3-amino-isoindolone et d'au moins une amine cationique hétérocyclique choisi parmi les composés de formule (I) suivante : 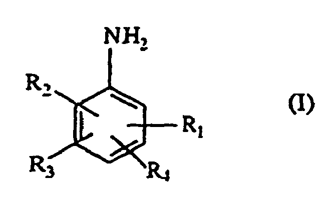 dans laquelle:
• R₁, R₂, R₃, R₄, identiques ou différents, représentent un atome d'hydrogène ; un atome d'halogène, un groupement -NH₂, un groupement -OH, ; un groupement Z ; un groupe -COZ ; un groupe -COOZ; un radical alkyle carbonyle ; un radical aminoalkyle carbonyle ; un radical N-alkylaminoalkyle carbonyle ; un radical N,N-dialkylaminoalkyle carbonyle ; un radical aminoalkyle carbonylalkyle ; un radical N-alkylaminoalkyle carbonylalkyle ; un radical N,N-dialkylaminoalkyle carbonylalkyle ; un radical carboxy ; un radical alkylcarboxy ; un radical alkylsulfonyle ; un radical aminosulfonyle ; un radical N-alkylaminosulfonyle ; un radical N,N-dialkylaminosulfonyle ; un radical aminosulfonalkyle ; un radical N-alkyl aminosulfonylalkyle ; un radical N,N-dialkyl aminosulfonylalkyle ; un radical carbamyle ; un radical N-alkyl carbamyle ; un radical N,N-dialkylcarbamyle ; un radical carbamylalkyle ; un radical N-alkyl carbamylalkyle ; un radical N,N-dialkyl carbamylalkyle ; un radical alkyle, monohydroxyalkyle, polyhydroxyalkyle, alcoxyalkyle, trifluoroalkyle, un radical cyano ; un groupement ORᵢ, SRᵢ, ORᵢZ ou SRᵢZ ou un groupe amino protégé par un radical alkylcarboxy, trifluoroalkylcarbonyle, aminoalkylcarbonyle, carbonyle, N-alkylaminoalkylcarbonyle, N,N-dialkylaminoalkyl-carbonyle, alkylcarboxy, carbamyle, N-alkylcarbamyle, N,N-dialkylcarbamyle, alkylsulfonyle, aminosulfonyle, N-alkylaminosulfonyle, N,N-dialkylaminosulfonyle, thiocarbamyle, formyle, un groupe -COZ ou un groupe -COOZ ;
• Rᵢ désigne un radical alkyle, monohydroxyalkyle, polyhydroxyalkyle, un groupement Z, un radical alcoxyalkyle ; un radical aryle ; un radical benzyle, un radical carboxyalkyle, un radical alkylcarboxyalkyle, un radical cyanoalkyle, un radical carbamylalkyle, un radical N-alkylcarbamylalkyle ; un radical N,N-dialkylcarbamylakyle ; un radical trifluoroalkyle ; un radical aminosulfonylalkyle ; un radical N-alkylaminosulfonylalkyle ; un radical N,N-dialkylaminosulfonyl-alkyle ; un radical alkylsulfinylalkyle ; un radical alkylsulfonyl-alkyle ; un radical alkylcarbonylalkyle ; un radical aminoalkyle ; un radical aminoalkyle dont l'amine est substituée par un ou deux radicaux identiques ou différents choisis parmi les radicaux alkyle, monohydroxyalkyle ; polyhydroxyalkyle, alkylcarbonyle, formyle, trifluoroalkylcarbonyle, alkylcarboxy, carbamyle, N-alkylcarbamyle, N,N-dialkylcarbamyle, thiocarbamyle, alkyl-sulfonyle et parmi les groupes Z, -COZ, ou -COOZ ;
Z représentant un groupement de formules (II) ou (III) suivante : 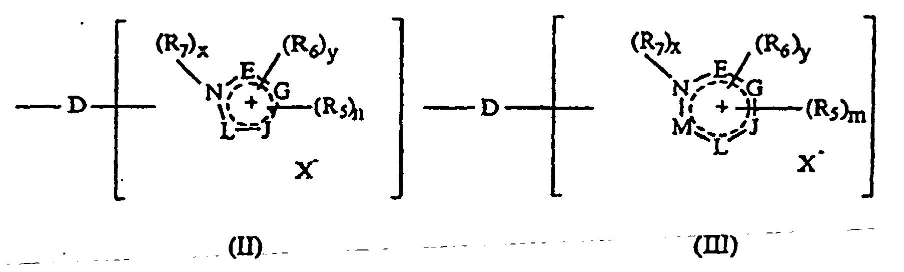 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone;
• les sommets E, G, J, L et M, identiques ou différents, représentent un atome de carbone, d'oxygène, de soufre ou d'azote;
• n est un nombre entier compris entre 0 et 4 inclusivement;
• m est un nombre entier compris entre 0 et 5 inclusivement;
• les radicaux R₅, identiques ou différents, représentent l'une des deux valences d'un bras de liaison D, un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁₋C₆, un radical alkyl(C₁-C₆)thio, un radical amino, un radical amino protégé par un radical alkyl(C₁-C₆)carbonyle, carbamyle ou alkyl(C₁₋C₆)sulfonyle; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆;
• R₆ représente l'une des deux valences d'un bras de liaison D, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁₋C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z;
• R₇ représente l'une des deux valences d'un bras de liaison D. un radical alkyle en C₁-C₆; un radical monohydroxyalkyle en C₁-C₆; un radical polyhydroxyalkyle en C₂-C₆; un radical aryle; un radical benzyle; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁₋C₆; un radical cyanoalkyle en C₁-C₆; un radical carbamylalkyle en C₁₋C₆; un radical trifluoroalkyle en C₁-C₆; un radical trialkyl(C₁₋C₆)silanealkyle en C₁-C₆; un radical sulfonamidoalkyle en C₁-C₆; un radical alkyl(C₁-C₆)carboxyalkyle en C₁-C₆; un radical alkyl(C₁₋C₆)sulfinylalkyle en C₁-C₆; un radical alkyl(C₁-C₆)sulfonylalkyle en C₁-C₆; un radical alkyl(C₁-C₆)cétoalkyle en C₁-C₆; un radical N-alkyl(C₁-C₆)carbamylalkyle en C₁-C₆; un radical N-alkyl(C₁₋C₆)sulfonamidoalkyle en C₁-C₆;
• x et y sont des nombres entiers égaux à 0 ou 1; avec les conditions suivantes:
- (i) dans les groupements cationiques insaturés de formule (II):
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que:
1) lorsque les sommets E, G, J et L représentent simultanément un atome de carbone, et que.le radical R₆ est porté par l'atome d'azote du cycle insaturé ; ou bien
2) lorsqu'au moins un des sommets E, G, J et L représente un atome d'azote sur lequel le radical R₆ est fixé;
- (ii) dans les groupements cationiques insaturés de formule (III):
- lorsque x = 0, le bras de liaison D est rattaché à l'atome d'azote,
- lorsque x = 1, le bras de liaison D est rattaché à l'un des sommets E, G, J. Lou M,
- y ne peut prendre la valeur 1 que lorsqu'au moins un des sommets E, G, J, L et M représente un atome divalent, et que le radical R₆ est porté par l'atome d'azote du cycle insaturé;
• X⁻ représente un anion monovalent ou divalent, et représente de préférence un atome d'halogène tel que le chlore, le brome, le fluor ou l'iode, un hydroxyde, un hydrogènesulfate, ou un alkylsulfate tel que par exemple un méthylsulfate ou un éthylsulfate ; et
• le composé (I) défini ci-dessus présente au moins un groupement Z.
, permettant d'obtenir, par réaction sans agent oxydant une coloration desdites fibres kératiniques.

2. Utilisation selon la revendication 1, **caractérisée en ce que** l'aldéhyde correspond à la formule (IV) suivante: 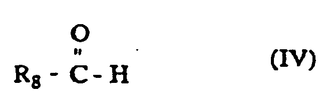 dans laquelle :
R₈ désigne un groupement de formule (IV A) suivante: 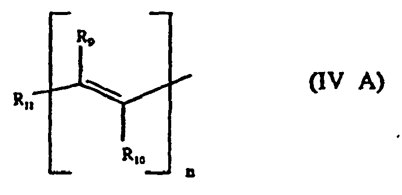
dans laquelle :
R₉ et R₁₀, identiques ou différents, désignent un atome d'hydrogène, un groupement alkyle, mono ou polyhydroxyalkyle, alkylhydroxyalkyle, alcoxy, -CF₃ ou -OCF₃,
R₉ et R₁₀ peuvent également former conjointement avec les atomes auxquels ils sont rattachés un cycle aryle ou un hétérocyclique à 5 ou 6 chaînons, lesdits cycles pouvant être substitués ou non;
n désigne un nombre entier de 0 à 3,
R₁₁ désigne les substituants désignés par R₉, un groupement aryle, alkylaryle substitué ou non, un groupe hétérocyclique à 5 ou 6 chaînons substitué ou non,
ou aux sels cosmétiquement acceptables de ces composés.

3. Utilisation selon la revendication 1 ou 2, **caractérisée en ce que** la cétone correspond aux formules (V) ou (VI) suivantes : 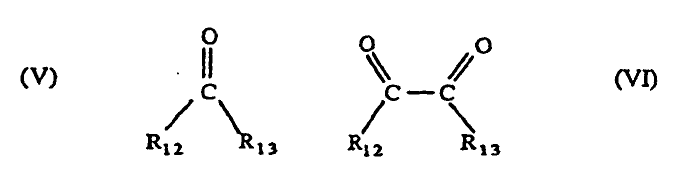 dans lesquelles :
R₁₂ désigne les substituants désignés par R₉,
R₁₃ désigne un groupement alkyle, mono ou polyhydroxyalkyle, alkylhydroxyalkyle, un groupement aryle, alkylaryle, un hétérocyclique à 5 ou 6 chaînons substitué ou non,
R₁₂ et. R₁₃ peuvent également former conjointement avec les atomes auxquels ils sont rattachés un cycle aryle à 5 ou 6 chaînons, ou un hétérocyclique comprenant des hétéroatomes tels que N ou S, ledit cycle pouvant lui-même être rattaché à un cycle aryle à 5 ou 6 chaînons ou à un hétérocycle comprenant des hétéroatomes tels que N ou S, lesdits cycles pouvant être substitués ou non,
ou aux sels cosmétiquement acceptables de ces composés.

4. Utilisation selon l'une quelconque des revendications 1 à 3, **caractérisée en ce que** la quinone correspond aux formules (VII) et (VIII) suivantes : 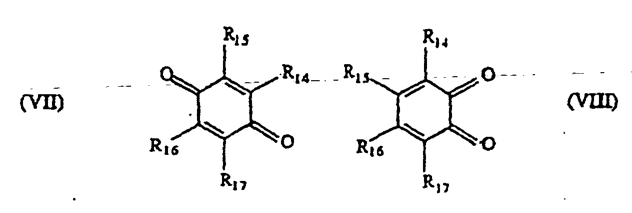 dans lesquelles :
R₁₄ désigne un atome d'hydrogène, d'halogène, un groupement sulfonique ou alcoxy.
R₁₅, R₁₆ et R₁₇, identiques ou différents désignent un atome d'hydrogène, d'halogène, un groupement hydroxy, alkyle, mono ou polyhydroxyalkyle, alkylhydroxyalkyle, alkylsulfonyle, carboxyalkyle, aminoalkyle, alkylaminoalkyle, (di-hydroxy)alkylaminoalkyle, ou alkyle-NR'R" (avec R' et R" désignant alkyle ou pouvant former ensemble avec l'atome d'azote auxquels ils sont rattachés un cycle aryle ou un hétérocycle à 5 ou 6 chaînons), un groupement aryle, un groupe amino pouvant être substitué par un alkyle ou un hydroxyalkyle,
R₁₄ et R₁₅, R₁₅ et R₁₆ ou R₁₆ et R₁₇ peuvent former conjointement avec les atomes auxquels ils sont rattachés un cycle aryle ou un hétérocycle à 5 ou 6 chaînons, substitué ou non;
ou aux sels cosmétiquement acceptables de ces composés.

5. Utilisation selon l'une quelconque des revendications 1 à 4, **caractérisée en ce que** les dérivés de la di-imino-isoindoline ou de la 3-amino-isoindolone correspondent à la formule (IX) suivante : 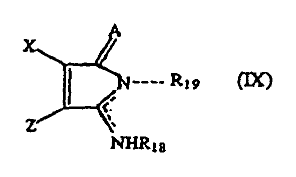 dans laquelle :
R₁₈ et R₁₉, identiques ou différents, désignent un atome d'hydrogène, un groupement alkyle, mono ou polyhydroxyalkyle, alkylhydroxyalkyle, aminoalkyle, alkylaminoalkyle, (dihydroxy)alkylarninoalkyle, ou un groupement alkyle NR'R", avec R' et R" désignant alkyle ou pouvant former conjointement avec l'atome d'azote auxquels ils sont rattachés un cycle aryle ou un hétérocycle à 5 ou 6 chaînons).
A désigne un atome d'oxynène ou NH,
X et Z forment ensemble un cycle aryle ou un hétérocycle à 5 ou 6 chaînons, substitué ou non;
ou aux sels cosmétiquement acceptables de ces composés.

6. Composition de teinture des fibres kératiniques, en particulier des fibres kératiniques humaines telles que les cheveux, **caractérisée par le fait qu'**elle comprend au moins une amine cationique hétérocyclique telle que définie dans la revendication 1 et au moins un composé choisi parmi un aldéhyde, une cétone, une quinone, et un dérivé de la diiminoisoindoline ou de la 3-amino isoindolone dans un milieu approprié pour la teinture, permettant d'obtenir, sans agent oxydant, une teinture desdites fibres kératiniques.

7. Composition de teinture selon la revendication 6 **caractérisée par le fait que** le composé choisi parmi un aldéhyde, une cétone, une quinone et un dérivé de la di-imino-isoindoline ou de la 3-amino-isoindolone est choisi parmi les composés définis selon les revendications 2 à 5.

8. Composition de teinture selon la revendication 6 ou 7, **caractérisée par le fait qu'**elle a un pH compris entre 2 et 11.

9. Composition selon l'une quelconque des revendications 6 à 8, **caractérisée par le fait que** l'amine cationique hétérocyclique est présente dans une concentration allant de 0,1 à 10 % et de préférence de 0,5 à 5 % en poids par rapport au poids total de la composition.

10. Composition selon l'une quelconque des revendications 6 à 9, **caractérisée par le fait que** le composé choisi parmi un aldéhyde, une cétone, une quinone et un dérivé de la di-imino-isoindoline ou de la 3-amino-isoindolone est présent dans une concentration allant de 0,1 à 10 % et de préférence de 0,5 à 5 % en poids par rapport au poids total de la composition.

11. Composition selon l'une quelconque des revendications 6 à 10, **caractérisée par le fait que** le milieu approprié pour la teinture est un milieu aqueux constitué par de l'eau et/ou des solvants organiques choisis parmi les alcools, les glycols et les éthers de glycol, dans des proportions comprises entre 0,5 et 20% en poids par rapport au poids total de la composition.

12. Procédé de teinture des fibres kératiniques, en particulier des fibres kératiniques humaines telles que les cheveux, **caractérisé par le fait qu'**il consiste à appliquer sur lesdites fibres un composant (A) constitué d'une composition renfermant dans un milieu approprié pour la teinture, au moins une amine cationique hétérocyclique telle que définie dans la revendication 1 et au moins un composant (B) constitué d'une composition contenant dans un milieu approprié pour la teinture, au moins un composé choisi parmi un aldehyde, une cétone, une quinone et un dérivé de la di-imino-isoindoline ou de la 3-amino-isoindolone de façon à permettre le développement d'une teinture avec lesdites fibres kératiniques par réaction sans agent oxydant.

13. Procédé selon la revendication 12, **caractérisé par le fait que** le composé choisi parmi un aldéhyde, une cétone, une quinone et un dérivé de la di-imino-isoindoline ou de la 3-amino-isoindolone est choisi parmi les composés selon l'une quelconque des revendications 2 à 5.

14. Procédé selon la revendication 12 or 13, **caractérisé par le fait qu'**il consiste à mélanger les composants (A) ou (B) juste avant emploi, à appliquer immédiatement la composition résultante sur les fibres kératiniques et à laisser agir pendant 1 à 60 minutes et préférentiellement pendant 1 à 30 minutes ; les fibres kératiniques étant ensuite rincées, lavées au shampooing, rincées à nouveau, puis séchées.

15. Procédé selon la revendication 12 ou 13, **caractérisé par le fait qu'**il consiste à appliquer sur les fibres kératiniques le composant (A), suivie ou précédée de l'application sur lesdites fibres du composant (B). à laisser agir chaque composant pendant 1 à 60 minutes et préférentiellement pendant 1 à 30 minutes, à procéder éventuellement au rinçage à l'eau entre chaque application; les fibres kératiniques étant ensuite rincées, lavées an shamponing, rincées à nouveau, puis séchées.

16. Agent de teinture des fibres kératiniques, et en particulier des fibres kératiniques humaines telles que les cheveux, **caractérisé par le fait qu'**il comporte les composants (A) et (B) tels que définis dans les revendications 12 à 15, sous forme séparée; les composants (A) et (B) étant destinés à être, soit mélangés tout juste avant emploi, soit appliqués de façon successive sur les fibres à traiter.

17. Dispositif à plusieurs compartiments ou "kit de teinture", **caractérisé par le fait qu'**il comporte au moins deux compartiments dont un renferme le composant (A) tel que défini dans la revendication 12, et le second renferme le composant (B) tel que défini dans la revendication 12 ou 13.

18. Dispositif selon la revendication 17, **caractérisé par le fait que** le composant (A) et/ou le composant (B) se présente(nt) sous forme de composition anhydre et qu'il comporte un troisième compartiment contenant un milieu aqueux cosmétiquement acceptable approprié pour la teinture destiné à être mélangé avant emploi dans l'un ou les deux premiers compartiments renfermant chaque composant (A) ou (B).

## Claims

1. Use, for dyeing keratin fibres, in particular human keratin fibres such as human hair, of at least one compound chosen from an aldehyde, a ketone, a quinone and a diiminoisoindoline or 3-aminoisoindolone derivative and of at least one heterocyclic cationic amine chosen from the compounds of formula (I) below: 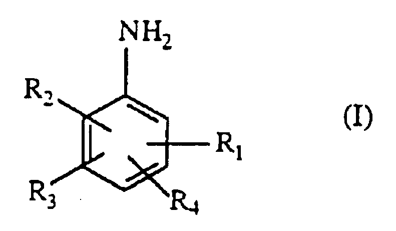 in which:
• R₁, R₂, R₃ and R₄, which may be identical or different, represent a hydrogen atom; a halogen atom, an -NH₂ group, an -OH group; a group Z; a group -COZ; a group -COOZ; an alkylcarbonyl radical; an aminoalkylcarbonyl radical; an N-alkylaminoalkylcarbonyl radical; an N,N-dialkylaminoalkylcarbonyl radical; an aminoalkylcarbonylalkyl radical; an N-alkylaminoalkylcarbonylalkyl radical; an N,N-dialkylaminoalkylcarbonylalkyl radical; a carboxyl radical; an alkylcarboxyl radical; an alkylsulphonyl radical; an aminosulphonyl radical; an N-alkylaminosulphonyl radical; an N,N-dialkylaminosulphonyl radical; an aminosulphonylalkyl radical; an N-alkylaminosulphonylalkyl radical; an N,N-dialkylaminosulphonylalkyl radical; a carbamyl radical; an N-alkylcarbamyl radical; an N,N-dialkylcarbamyl radical; a carbamylalkyl radical; an N-alkylcarbamylalkyl radical; an N,N-dialkylcarbamylalkyl radical; an alkyl, monohydroxyalkyl, polyhydroxyalkyl, alkoxyalkyl or trifluoroalkyl radical or a cyano radical; a group ORᵢ, SRᵢ, ORᵢZ or SRᵢZ or an amino group protected with an alkylcarboxyl, trifluoroalkylcarbonyl, aminoalkylcarbonyl, carbonyl, N-alkylaminoalkylcarbonyl, N,N-di-alkylaminoalkylcarbonyl, alkylcarboxyl, carbamyl, N-alkylcarbamyl, N,N-dialkylcarbamyl, alkylsulphonyl, aminosulphonyl, N-alkylaminosulphonyl, N,N-dialkylaminosulphonyl, thiocarbamyl or formyl radical, a group -COZ or a group -COOZ;
• Rᵢ denotes an alkyl, monohydroxyalkyl or polyhydroxyalkyl radical, a group Z, an alkoxyalkyl radical; an aryl radical; a benzyl radical, a carboxyalkyl radical, an alkylcarboxyalkyl radical, a cyanoalkyl radical, a carbamylalkyl radical or an N-alkylcarbamylalkyl radical; an N,N-dialkylcarbamylalkyl radical; a trifluoroalkyl radical; an aminosulphonylalkyl radical; an N-alkylaminosulphonylalkyl radical; an N,N-dialkylaminosulphonylalkyl radical; an alkylsulphinylalkyl radical; an alkylsulphonylalkyl radical; an alkylcarbonylalkyl radical; an aminoalkyl radical; an aminoalkyl radical in which the amine is substituted with one or two radicals, which may be identical or different, chosen from alkyl, monohydroxyalkyl, polyhydroxyalkyl, alkylcarbonyl, formyl, trifluoroalkylcarbonyl, alkylcarboxyl, carbamyl, N-alkylcarbamyl, N,N-dialkylcarbamyl, thiocarbamyl and alkylsulphonyl radicals and from the groups Z, -COZ or -COOZ;
Z representing a group of formula (II) or (III) below: 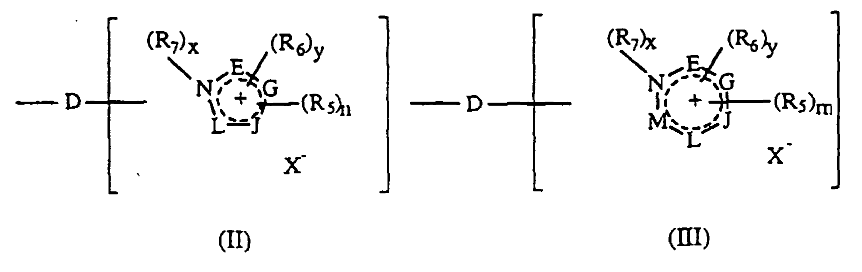 in which:
• D is a linker arm which represents a linear or branched alkyl chain preferably containing from 1 to 14 carbon atoms, which can be interrupted by one or more hetero atoms such as oxygen, sulphur or nitrogen atoms, and which can be substituted with one or more hydroxyl or C₁-C₆ alkoxy radicals, and which can bear one or more ketone functions;
• the ring members E, G, J, L and M, which may be identical or different, represent a carbon, oxygen, sulphur or nitrogen atom;
• n is an integer between 0 and 4 inclusive;
• m is an integer between 0 and 5 inclusive;
• the radicals R₅, which may be identical or different, represent one of the two valencies of a linker arm D, a second group Z which is identical to or different from the first group Z, a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆) alkyl radical, a C₁-C₆ alkoxy radical, a tri(C₁-C₆)alkylsilane(C₁-C₆)alkyl radical, an amido radical, an aldehydo radical, a carboxyl radical, a (C₁-C₆)alkylcarbonyl radical, a thio radical, a C₁-C₆ thioalkyl radical, a C₁-C₆ alkylthio radical, an amino radical, an amino radical protected with a (C₁-C₆) alkylcarbonyl, carbamyl or C₁-C₆ alkylsulphonyl radical; a group NHR" or NR"R"' in which R" and R"', which may be identical or different, represent a C₁-C₆ alkyl radical, a C₁-C₆ monohydroxyalkyl radical or a C₂-C₆ polyhydroxyalkyl radical;
• R₆ represents one of the two valencies of a linker arm D, a C₁-C₆ alkyl radical, a C₁-C₆ monohydroxyalkyl radical, a C₂-C₆ polyhydroxyalkyl radical, a cyano-(C₁-C₆) alkyl radical, a tri (C₁-C₆) alkylsilane (C₁-C₆) alkyl radical, a (C₁-C₆) alkoxy(C₁-C₆)alkyl radical, a carbamyl (C₁-C₆) alkyl radical, a (C₁-C₆)alkylcarboxy-(C₁-C₆)alkyl radical, a benzyl radical, a second group Z which is identical to or different from the first group Z;
• R₇ represents one of the two valencies of a linker arm D, a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆) alkylcarbonyl, carbamyl or C₁-C₆ alkylsulphonyl radical; a carboxy(C₁-C₆)alkyl radical; a cyano (C₁-C₆) alkyl radical; a carbamyl (C₁-C₆) alkyl radical; a C₁-C₆ trifluoroalkyl radical; a tri(C₁-C₆)-alkylsilane(C₁-C₆)alkyl radical; a C₁-C₆ sulphonamidoalkyl radical; a (C₁-C₆) alkylcarboxy(C₁-C₆)alkyl radical; a (C₁-C₆) alkylsulphinyl (C₁-C₆) alkyl radical; a (C₁-C₆)alkylsulphonyl(C₁-C₆)alkyl radical; a (C₁-C₆)-alkylketo(C₁-C₆)alkyl radical; an N- (C₁-C₆) alkylcarbamyl(C₁-C₆)alkyl radical; an N-(C₁-C₆)alkylsulphonamido(C₁-C₆)alkyl radical;
• x and y are integers equal to 0 or 1; with the following conditions:
- (i) in the unsaturated cationic groups of formula (II) :
- when x = 0, the linker arm D is attached to the nitrogen atom,
- when x = 1, the linker arm D is attached to one of the ring members E, G, J or L,
- y can take the value 1 only:
1) when the ring members E, G, J and L simultaneously represent a carbon atom and when the radical R₆ is borne by the nitrogen atom of the unsaturated ring; or alternatively
2) when at least one of the ring members E, G, J and L represents a nitrogen atom to which the radical R₆ is attached;
- (ii) in the unsaturated cationic groups of formula (III) :
- when x = 0, the linker arm D is attached to the nitrogen atom,
- when x = 1, the linker arm D is attached to one of the ring members E, G, J, L or M,
- y can take the value 1 only when at least one of the ring members E, G, J, L and M represents a divalent atom and when the radical R₆ is borne by the nitrogen atom of the unsaturated ring;
• X⁻ represents a monovalent or divalent anion and preferably represents a halogen atom such as chlorine, bromine, fluorine or iodine, a hydroxide, a hydrogenosulphate or an alkyl sulphate such as, for example, a methyl sulphate or an ethyl sulphate; and
• the compound (I) defined above contains at least one group Z; in order to obtain, by reaction without an oxidizing agent, a coloration of the said keratin fibres.

2. Use according to Claim 1, **characterized in that** the aldehyde corresponds to formula (IV) below: 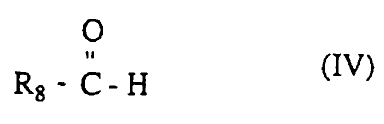 in which:
R₈ denotes a group of formula (IV A) below: 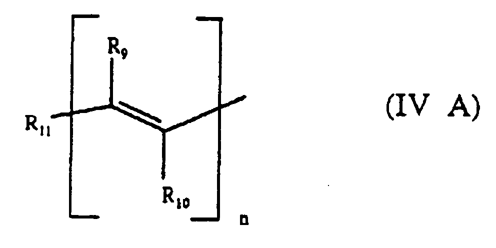
in which:
R₉ and R₁₀, which may be identical or different, denote a hydrogen atom or an alkyl, mono- or polyhydroxyalkyl, alkylhydroxyalkyl, alkoxy, -CF₃ or -OCF₃ group,
R₉ and R₁₀ can also form, together with the atoms to which they are attached, an aryl ring or a 5- or 6-membered heterocyclic ring, it being possible for the said rings to be substituted or unsubstituted;
n denotes an integer from 0 to 3,
R₁₁ denotes the substituents denoted by R₉, a substituted or unsubstituted aryl or alkylaryl group or a substituted or unsubstituted 5- or 6-membered heterocyclic group,
or to the cosmetically acceptable salts of these compounds.

3. Use according to Claim 1 or 2, **characterized in that** the ketone corresponds to formula (V) or (VI) below: 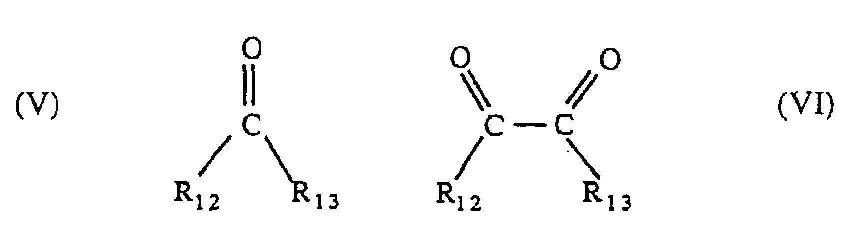 in which:
R₁₂ denotes the substituents denoted by R₉,
R₁₃ denotes an alkyl, mono- or polyhydroxyalkyl or alkylhydroxyalkyl group, or a substituted or unsubstituted aryl, alkylaryl or 5- or 6-membered heterocyclic group,
R₁₂ and R₁₃ can also form, together with the atoms to which they are attached, a 5- or 6-membered aryl ring or a heterocyclic ring comprising hetero atoms such as N or S, it being possible for the said ring itself to be attached to a 5- or 6-membered aryl ring or to a heterocycle comprising hetero atoms such as N or S, it being possible for the said rings to be substituted or unsubstituted,
or to the cosmetically acceptable salts of these compounds.

4. Use according to any one of Claims 1 to 3, **characterized in that** the quinone corresponds to formulae (VII) and (VIII) below: 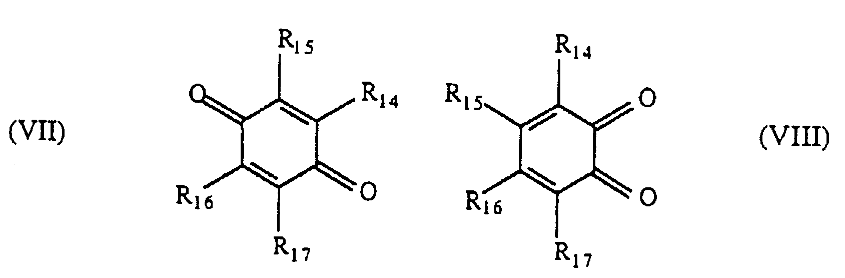 in which:
R₁₄ denotes a hydrogen or halogen atom or a sulphonic or alkoxy group,
R₁₅, R₁₆ and R₁₇, which may be identical or different, denote a hydrogen or halogen atom, a hydroxyl, alkyl, mono- or polyhydroxyalkyl, alkylhydroxyalkyl, alkylsulphonyl, carboxyalkyl, aminoalkyl, alkylaminoalkyl, (dihydroxy)alkylaminoalkyl or alkyl-NR'R" group (with R' and R" denoting alkyl or possibly forming, together with the nitrogen atom to which they are attached, an aryl ring or a 5- or 6-membered heterocycle), an aryl group or an amino group which can be substituted with an alkyl or a hydroxyalkyl,
R₁₄ and R₁₅, R₁₅ and R₁₆ or R₁₆ and R₁₇ can form, together with the atoms to which they are attached, a substituted or unsubstituted aryl ring or 5- or 6-membered heterocycle;
or to the cosmetically acceptable salts of these compounds.

5. Use according to any one of Claims 1 to 4, **characterized in that** the diiminoisoindoline or 3-aminoisoindolone derivatives correspond to formula (IX) below: 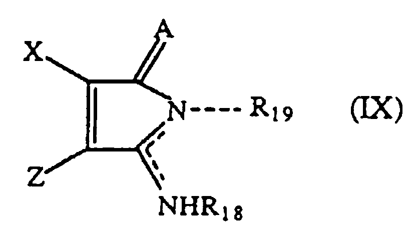 in which:
R₁₈ and R₁₉, which may be identical or different, denote a hydrogen atom, an alkyl, mono- or polyhydroxyalkyl, alkylhydroxyalkyl, aminoalkyl, alkylaminoalkyl or (dihydroxy)alkylaminoalkyl group or an alkyl NR'R" group, with R' and R" denoting alkyl or possibly forming, together with the nitrogen atom to which they are attached, an aryl ring or a 5- or 6-membered heterocycle),
A denotes an oxygen atom or NH,
X and Z together form a substituted or unsubstituted aryl ring or 5- or 6-membered heterocycle;
or to the cosmetically acceptable salts of these compounds.

6. Composition for dyeing keratin fibres, in particular human keratin fibres such as the hair, **characterized in that** it comprises at least one heterocyclic cationic amine as defined in Claim 1 and at least one compound chosen from an aldehyde, a ketone, a quinone and a diiminoisoindoline or 3-aminoisoindolone derivative in a medium which is suitable for dyeing, in order to obtain, without an oxidizing agent, a coloration of the said keratin fibres.

7. Dye composition according to Claim 6, **characterized in that** the compound chosen from an aldehyde, a ketone, a quinone and a diiminoisoindoline or 3-aminoisoindolone derivative is chosen from the compounds defined according to Claims 2 to 5.

8. Dye composition according to Claim 6 or 7, **characterized in that** it has a pH of between 2 and 11.

9. Composition according to any one of Claims 6 to 8, **characterized in that** the heterocyclic cationic amine is present in a concentration ranging from 0.1% to 10% and preferably from 0.5% to 5% by weight relative to the total weight of the composition.

10. Composition according to any one of Claims 6 to 9, **characterized in that** the compound chosen from an aldehyde, a ketone, a quinone and a diiminoisoindoline or 3-aminoisoindolone derivative is present in a concentration ranging from 0.1% to 10% and preferably from 0.5% to 5% by weight relative to the total weight of the composition.

11. Composition according to any one of Claims 6 to 10, **characterized in that** the medium which is suitable for dyeing is an aqueous medium consisting of water and/or organic solvents chosen from alcohols, glycols and glycol ethers, in proportions of between 0.5% and 20% by weight relative to the total weight of the composition.

12. Process for dyeing keratin fibres, in particular human keratin fibres such as the hair, **characterized in that** it consists in applying a component (A) consisting of a composition containing, in a medium which is suitable for dyeing, at least one heterocyclic cationic amine as defined in Claim 1, and at least one component (B) consisting of a composition containing, in a medium which is suitable for dyeing, at least one compound chosen from an aldehyde, a ketone, a quinone and a diiminoisoindoline or 3-aminoisoindolone derivative, to the said fibres so as to allow the development of a coloration with the said keratin fibres, by reaction without an oxidizing agent.

13. Process according to Claim 12, **characterized in that** the compound chosen from an aldehyde, a ketone, a quinone and a diiminoisoindoline or 3-aminoisoindolone derivative is chosen from the compounds according to any one of Claims 2 to 5.

14. Process according to Claim 12 or 13, **characterized in that** it consists in mixing components (A) and (B) just before use, in immediately applying the resulting composition to the keratin fibres and in leaving the composition to act for 1 to 60 minutes and preferably 1 to 30 minutes; the keratin fibres then being rinsed, washed with shampoo, rinsed again and then dried.

15. Process according to Claim 12 or 13, **characterized in that** it consists in applying component (A) to the keratin fibres, followed or preceded by application of component (B) to the said fibres, in leaving each component to act for 1 to 60 minutes and preferably 1 to 30 minutes, and optionally in rinsing with water between each application; the keratin fibres then being rinsed, washed with shampoo, rinsed again and then dried.

16. Agent for dyeing keratin fibres, and in particular human keratin fibres such as the hair, **characterized in that** it separately comprises components (A) and (B) as defined in Claims 12 to 15; the components (A) and (B) being intended to be either mixed immediately before use or applied successively to the fibres to be treated.

17. Multi-compartment device or "dyeing kit", **characterized in that** it comprises at least two compartments, one of which contains component (A) as defined in Claim 12, and the second of which contains component (B) as defined in Claim 12 or 13.

18. Device according to Claim 17, **characterized in that** component A and/or component B is (are) in the form of an anhydrous composition, and **in that** it comprises a third compartment containing a cosmetically acceptable aqueous medium which is suitable for dyeing and which is intended to be mixed, before use, into one or both of the first compartments containing each component (A) or (B).

## Patentansprüche

1. Verwendung zum Färben von Keratinfasern, insbesondere menschlichen Keratinfasern wie menschlichem Haar, mittels einer Verbindung aus der Reihe Aldehyd, Keton, Chinon sowie Diiminoisoindolin- oder 3-Aminoisoindolonderivat und mindestens einem heterocyclischen kationischen Amin aus der Reihe der Verbindungen der folgenden Formel (I) : 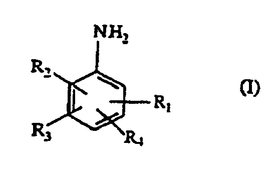 in der:
• R₁, R₂, R₃ und R₄, die gleich oder verschieden sind, folgende Bedeutungen aufweisen: Wasserstoffatom; Halogenatom; -NH₂-Gruppe; -OH-Gruppe; Z-Gruppe; -COZ-Gruppe; -COOZ-Gruppe; Alkylcarbonylrest; Aminoalkylcarbonylrest; N-Alkylaminoalkylcarbonylrest; N,N-Dialkylaminoalkylcarbonylalkylrest; Aminoalkylcarbonylalkylrest; N-Alkylaminoalkylcarbonylalkylrest; N,N-Dialkylaminoalkycarbonylalkylrest; Carboxyrest; Alkylcarboxyrest; Alkylsulfonylrest; Aminosulfonylrest; N-Alkylaminosulfonylrest; N,N-Dialkylaminosulfonylrest; Aminosulfonalkylrest; N-Alkylaminosulfonylalkylrest; N,N-Dialkylaminosulfonylalkylrest; Carbamylrest; N-Alkylcarbamylrest; N,N-Dialkylcarbamylrest; Carbamylalkylrest; N-Alyklcarbamylalkylrest; N,N-Dialkylcarbamylalkylrest; Alkyl-, Monohydroxyalkyl-, Polyhydroxyalkyl-, Alkoxyalkyl-, Trifluoralkylrest, Cyanorest; ORi-, SRi-, ORᵢZ- oder SRiZ-Gruppe oder Aminogruppe, die durch einen Alkylcarboxy-, Trifluoralkylcarbonyl-, Aminoalkylcarbonyl-, Carbonyl-, N-Alkylaminoalkylcarbonyl-, N,N-Dialkylaminoalkyl-carbonyl-, Alkylcarboxy-, Carbamyl-, N-Alkylcarbamyl-, N,N-Dialkylcarbamyl-, Alkylsulfonyl-, Aminosulfonyl-, N-Alkylaminosulfonyl-, N,N-Dialkylaminosulfonyl-, Thiocarbamyl- oder Formylrest oder eine -COZ-Gruppe oder eine -COOZ-Gruppe geschützt ist;
• Rᵢ die folgenden Bedeutungen aufweist: Alkyl-, Monohydrxyalkyl-, Polyhydroxyalkylrest, Z-Gruppe, Alkoxyalkylrest; Arylrest; Benzylrest, Carboxyalkylrest, Alkylcarboxyalkylrest, Cyanoalkylrest, Carbamylalkylrest, N-Alkylcarbamylalkylrest; N,N-Dialkylcarbamylalkylrest; Trifluoralkylrest; Aminosulfonylalkylrest; N-Alkylaminosulfonylalkylrest; N,N-Dialkylaminosulfonylalkylrest; Alkylsulfinylalkylrest; Alkylsulfonylalkylrest; Alkylcarbonylalkylrest; Aminoalkylrest; Aminoalkylrest, bei dem das Amin durch eine oder zwei gleiche oder verschiedene Reste aus der Gruppe Alkyl-, Monohydroxyalkyl-, Polyhydroxyalkyl-, Alkylcarbonyl-, Formyl-, Trifluoralkylcarbonyl-, Alkylcarboxy-, Carbamyl-, N-Alkylcarbamyl-, N,N-Dialkylcarbamyl-, Thiocarbamyl-, Alkylsulfonylrest und aus der Reihe der Gruppen Z, -COZ oder -COOZ substituiert ist;
wobei Z eine Gruppe der folgenden Formel (II) oder (III) bedeutet: 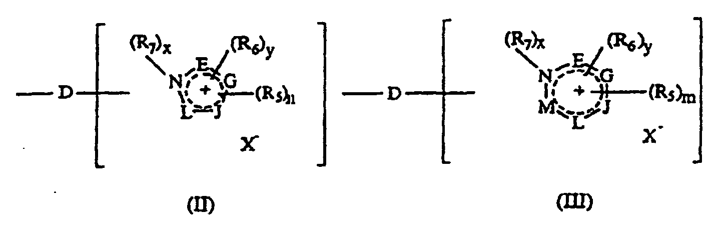 in denen:
• D ein Bindeglied darstellt, das eine geradkettige oder verzweigte Alkylkette mit vorzugsweise 1 bis 14 Kohlenstoffatomen, die durch ein oder mehrere Heteroatome wie Sauerstoff-, Schwefel- oder Stickstoffatome, unterbrochen sein kann und durch einen oder mehrere Hydroxy- oder C₁-C₆-Alkyoxyreste substituiert sein kann und eine oder mehrere Ketonfunktionen tragen kann, bedeutet;
• die Ringglieder E, G, J, L und M, die gleich oder verschieden sind, bedeuten ein Kohlenstoff-, Sauerstoff-, Schwefel- oder Stickstoffatom;
• n bedeutet eine ganze Zahl zwischen einschließlich 0 und 4;
• m bedeutet eine ganze Zahl zwischen einschließlich 0 und 5;
• die Reste R₅, die gleich oder verschieden sind, weisen die folgenden Bedeutungen auf: eine der beiden Valenzen eines Bindeglieds D, eine zweite Gruppe Z, die mit der ersten Gruppe Z identisch oder davon verschieden ist, Halogenatom, Hydroxyrest, C₁-C₆₋Alkylrest, C₁-C₆-Monohydroxyalkylrest, C₂-C₆₋Polyhydroxyalkylrest, Nitrorest, Cyanorest, C₁-C₆-Cyanoalkylrest, C₁-C₆-Alkoxyrest, Tri (C₁₋C₆)alkylsilan-C₁-C₆-alkylrest, Amidrest, Aldehydrest, Carboxyrest, C₁-C₆₋Alkylcarbonylrest, Thiorest, C₁-C₆₋Thioalkylrest, (C₁-C₆)Alkylthiorest, Aminorest, Aminorest, der durch einen (C₁₋C₆)Alkylcarbonyl-, Carbamyl- oder (C₁₋C₆)Alkylsulfonylrest geschützt ist; NHR"- oder NR"R"'-Gruppe, in der die Reste R" und R"', die gleich oder verschieden sind, einen C₁-C₆₋Alkylrest, C₁-C₆-Monohydroxyalkylrest oder C₂₋C₆-Polyhydroxyalkylrest bedeuten;
• R₆ die folgenden Bedeutungen aufweist: eine der beiden Valenzen eines Bindeglieds D, C₁₋C₆-Alkylrest, C₁-C₆-Monohydroxyalkylrest, C₂₋C₆-Polyhydroxyalkylrest, C₁-C₆-Cyanoalkylrest, Tri (C₁-C₆) alkylsilan-C₁-C₆-alkylrest, (C₁₋C₆)Alkoxy-(C₁-C₆)alkylrest, C₁-C₆-Carbamylalkylrest, (C₁-C₆)Alkylcarboxy-C₁-C₆-alkylrest, Benzylrest, sowie eine zweite Gruppe Z, die mit der ersten Gruppe Z identisch oder davon verschieden ist;
• R₇ die folgenden Bedeutungen aufweist: eine der beiden Valenzen eines Bindeglieds D, C₁₋C₆-Alkylrest, C₁-C₆-Monohydroxyalkylrest, C₂₋C₆-Polyhydroxyalkylrest; Arylrest; Benzylrest; C₁-C₆-Aminoalkylrest, C₁-C₆-Aminoalkylrest, wobei das Amin durch einen (C₁-C₆)Alkylcarbonyl-, Carbamyl- oder (C₁₋C₆)Alkylsulfonylrest geschützt ist; C₁-C₆₋Carboxyalkylrest; C₁-C₆-Cyanoalkylrest; C₁-C₆₋Carbamylalkylrest; C₁-C₆-Trifluoralkylrest; Tri (C₁-C₆) alkyl-silan-C₁-C₆-alkylrest; C₁-C₆₋Sulfonamidoalkylrest; (C₁-C₆) -Alkyl-C₁-C₆₋carboxyalkylrest; (C₁-C₆) -Alkyl-C₁-C₆₋sulfinylalkylrest; (C₁-C₆) -Alkyl-C₁-C₆₋sulfonylalkylrest; (C₁-C₆) -Alkyl-C₁-C₆₋ketoalkylrest; N-(C₁-C₆)-alkyl-C₁-C₆₋carbamylalkylrest; N-(C₁-C₆)-alkylsulfonamido-C₁-C₆-alkylrest;
• x und y ganze Zahlen gleich 0 oder 1 bedeuten, mit den folgenden Maßgaben:
- (i) in den ungesättigten kationischen Gruppen der Formel (II)
- ist, wenn x = 0, das Bindeglied D an das Stickstoffatom gebunden,
- wenn x = 1, das Bindeglied D an eines der Ringglieder E, G, J oder L gebunden;
- kann y dann nicht den Wert annehmen, wenn
1) die Ringglieder E, G, J und L gleichzeitig ein Kohlenstoffatom bedeuten und der Rest R₆ vom Stickstoffatom des ungesättigten Rings getragen wird; oder
2) mindestens eines der Ringglieder E, G, J und L ein Stickstoffatom bedeutet, an das der Rest R₆ gebunden ist;
- (ii)in den ungesättigten kationischen Gruppen der Formel (III)
- ist, wenn x = 0, das Bindeglied D an das Stickstoffatom gebunden,
- wenn x = 1, das Bindeglied D an eines der Ringglieder E, G, J, L oder M gebunden;
- kann y dann nicht den Wert 1 annehmen, wenn wenigstens eines der Ringglieder E, G, J, L und M ein zweiwertiges Atom bedeutet und der Rest R₆ vom Stickstoffatom des ungesättigten Rings getragen wird;
• X⁻ ein einwertiges oder zweiwertiges Anion, vorzugsweise ein Halogenatom wie Chlor, Brom, Fluor oder Iod, ein Hydroxid, Hydrogensulfat oder Alkylsulfat, wie zum Beispiel Methylsulfat oder Ethylsulfat bedeutet; und
• die oben definierte Verbindung (I) mindestens eine Gruppe Z aufweist, wodurch man mit einer Reaktion ohne Oxidationsmittel zu einer Färbung dieser Keratinfasern gelangt.

2. Verwendung nach Anspruch 1, **dadurch gekennzeichnet, daß** der Aldehyd der folgenden Formel (IV) : 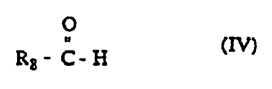 in der
R₈ eine Gruppe der folgenden Formel (IV A) bedeutet: 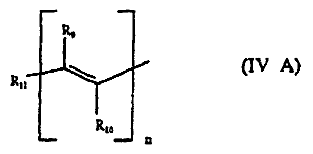 in der:
R₉ und R₁₀, die gleich oder verschieden sind, ein Wasserstoffatom oder eine Alkyl-, Mono- oder Polyhydroxyalkyl-, Alkylhydroxyalkyl-, Alkoxy-, -CF₃- oder -OCF₃-Gruppe bedeuten,
Rg und R₁₀ auch gemeinsam mit den Atomen, an die sie gebunden sind, einen Arylring oder einen 5-oder 6-gliedrigen Heterocyclus, wobei diese Ringe gegebenenfalls substituiert sein können, bilden können;
n eine ganze Zahl von 0 bis 3 bedeutet,
R₁₁ die durch R₉ bezeichneten Substituenten, eine Arylgruppe, eine gegebenenfalls substituierte Alkylarylgruppe, eine gegebenenfalls substituierte 5- oder 6-gliedrige heterocyclische Gruppe bedeutet, oder den kosmetisch annehmbaren Salzen dieser Verbindungen entspricht.

3. Verwendung nach Anspruch 1 oder 2, **dadurch gekennzeichnet, daß** das Keton den folgenden Formeln (V) oder (VI): 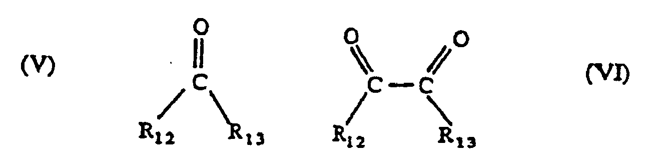 in denen:
R₁₂ die durch R₉ angegebenen Substituenten bedeutet,
R₁₃ eine Alkyl-, Mono- oder Polyhydroxyalkyl-, Alkylhydroxyalkylgruppe, Aryl-, Alkylarylgruppe, einen gegebenenfalls substituierten Heterocyclus mit 5 oder 6 Ringgliedern bedeutet,
R₁₂ und R₁₃ auch gemeinsam mit den Atomen, an die sie gebunden sind, einen Arylring mit 5 oder 6 Ringgliedern oder einen Heterocyclus mit Heteroatomen wie N oder S, wobei dieser Ring selbst wiederum an einen Arylring mit 5 oder 6 Ringgliedern oder an einen Heterocyclus mit Heteroatomen wie N oder S gebunden ist, wobei diese Ringe gegebenenfalls substituiert sein können, bilden können,
oder den kosmetisch annehmbaren Salzen dieser Verbindungen entspricht.

4. Verwendung nach einem der Ansprüche 1 bis 3, **dadurch gekennzeichnet, daß** das Chinon den folgenden Formeln (VII) und (VIII): 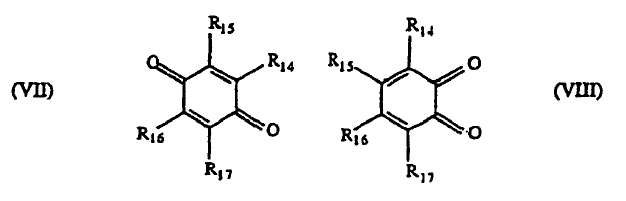 in denen:
R₁₄ ein Wasserstoff-, Halogenatom, eine Sulfon- oder Alkoxygruppe bedeutet,
R₁₅, R₁₆ und R₁₇, die gleich oder verschieden sind, ein Wasserstoff-, Halogenatom, eine Hydroxy-, Alkyl-, Mono- oder Polyhydroxyalkyl-, Alkylhydroxyalkyl-, Alkylsulfonyl-, Carboxyalkyl-, Aminoalkyl-, Alkylaminoalkyl-, (Dihydroxy)alkylaminolkyl- oder NR'R"-Alkyl (wobei R' und R" Alkyl bedeuten oder gemeinsam mit dem Stickstoffatom, an das sie gebunden sind, einen Aryl- oder Heterocyclus mit 5 oder 6 Ringgliedern bilden können), Arylgruppe, Aminogruppe, die durch ein Alkyl oder Hydroxyalkyl substituiert sein kann, bedeuten,
R₁₄ und R₁₅, R₁₅ und R₁₆ oder R₁₆ und R₁₇ gemeinsam mit den Atomen, an die sie gebunden sind, einen Arylring oder Heterocyclus mit 5 oder 6 Ringgliedern, der gegebenenfalls substituiert ist, bilden können;
oder den kosmetisch annehmbaren Salzen dieser Verbindungen entspricht.

5. Verwendung nach einem der Ansprüche 1 bis 4, **dadurch gekennzeichnet, daß** die Diiminoisoindolin- oder 3-Aminoisoindolonderivate der folgenden Formel (IX): 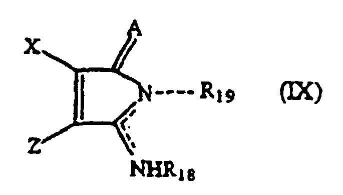 in der:
R₁₈ und R₁₉, die gleich oder verschieden sind, ein Wasserstoffatom, eine Alkyl-, Mono- oder Polyhydroxyalkyl-, Alkylhydroxyalkyl-, Aminoalkyl-, Alkylaminoalkyl-, (Dihydroxy)alkylaminolkyl- oder Alkyl-NR'R"-alkylgruppe, wobei R' und R" Alkyl bedeuten oder gemeinsam mit dem Stickstoffatom, an das sie gebunden sind, einen Arylring oder Heterocyclus mit 5 oder 6 Ringgliedern bilden können, bedeuten A ein Sauerstoffatom oder NH bedeutet,
X und Z gemeinsam einen Arylring oder einen Heterocyclus mit 5 oder 6 Ringgliedern, der gegebenenfalls substituiert ist, bilden;
oder den kosmetisch annehmbaren Salzen dieser Verbindungen entspricht.

6. Zusammensetzung zum Färben von Keratinfasern, insbesondere von menschlichen Keratinfasern wie dem Haar, **dadurch gekennzeichnet, daß** diese mindestens ein wie in Anspruch 1 definiertes kationisches heterocyclisches Amin sowie mindestens eine Verbindung aus der Reihe Aldehyd, Keton, Chinon sowie Diiminoisoindolin- oder 3-Aminoisoindolonderivat in einem zum Färben geeigneten Medium enthält, wodurch man ohne Oxidationsmittel zu einer Färbung dieser Keratinfasern gelangt.

7. Färbezusammensetzung nach Anspruch 6, **dadurch gekennzeichnet, daß** die Verbindung aus der Reihe Aldehyd, Keton, Chinon und Diiminoisoindolin- oder 3-Aminoisoindolonderivat aus der Reihe der gemäß Ansprüchen 2 bis 5 definierten Zusammensetzungen stammt.

8. Färbezusammensetzung nach Anspruch 6 oder 7 **dadurch gekennzeichnet, daß** sie einen pH-Wert zwischen 2 und 11 aufweist.

9. Zusammensetzung nach einem der Ansprüche 6 bis 8, **dadurch gekennzeichnet, daß** das kationische heterocyclische Amin in einer Konzentration von 0,1 bis 10 Gew.-%, vorzugsweise 0,5 bis 5 Gew.-%, in Bezug auf das Gesamtgewicht der Zusammensetzung vorliegt.

10. Zusammensetzung nach einem der Ansprüche 6 bis 9, **dadurch gekennzeichnet, daß** die Verbindung aus der Reihe Aldehyd, Keton, Chinon und Diiminoisoindolin- oder 3-Aminoisoindolonderivat in einer Konzentration von 0,1 bis 10 Gew.-%, vorzugsweise 0,5 bis 5 Gew.-%, in Bezug auf das Gesamtgewicht der Zusammensetzung vorliegt.

11. Zusammensetzung nach einen der Ansprüche 6 bis 10, **dadurch gekennzeichnet, daß** es sich bei dem für das Färben geeignete Medium um ein wäßriges Medium handelt, das aus Wasser und/oder organischen Lösungsmitteln aus der Reihe Alkohole, Glycole und Glycolether in Mengen zwischen 0,5 und 20 Gew.-%, in Bezug auf das Gesamtgewicht der Zusammensetzung besteht.

12. Verfahren zur Färbung von Keratinfasern, insbesondere menschlichen Keratinfasern wie dem Haar, **dadurch gekennzeichnet, daß** man dabei eine Komponente (A), die aus einer Zusammensetzung besteht, welche mindestens ein wie in Anspruch 1 definiertes kationisches heterocyclisches Amin in einem zum Färben geeigneten Medium umfaßt, sowie mindestens eine Komponente (B), die aus einer Zusammensetzung besteht, die mindestens eine Verbindung der Reihe Aldehyd, Keton, Chinon und Diiminoisoindolin- oder 3-Aminoisoindolonderivat in einem zum Färben geeigneten Medium enthält, auf diese Fasern so aufträgt, daß man ohne Reaktion mit einem Oxidationsmittel zur Entwicklung einer Färbung bei diesen Keratinfasern gelangt.

13. Verfahren nach Anspruch 12, **dadurch gekennzeichnet, daß** die Verbindung aus der Reihe Aldehyd, Keton, Chinon und Diiminoisoindolin- oder 3-Aminoisoindolonderivat aus der Reihe der Verbindungen nach einem der Ansprüche 2 bis 5 stammt.

14. Verfahren nach Anspruch 12 oder 13, **dadurch gekennzeichnet, daß** man dabei die Komponenten (A) und (B) unmittelbar vor Verwendung mischt und die erhaltene Zusammensetzung sofort auf die Keratinfasern aufträgt und 1 bis 60 Minuten, vorzugsweise 1 bis 30 Minuten, einwirken läßt, wobei die Keratinfasern anschließend gespült, mit Shampoo gewaschen, nochmals gespült und anschließend getrocknet werden.

15. Verfahren nach Anspruch 12 oder 13, **dadurch gekennzeichnet, daß** man dabei die Komponente (A) auf die Keratinfasern aufträgt und die Komponente (B) danach oder zuvor auf diese Fasern aufträgt, jede Komponente 1 bis 60 Minuten vorzugsweise 1 bis 30 Minuten einwirken läßt, und schließlich nach jedem Auftrag mit Wasser spült, wobei die Keratinfasern anschließend gespült, mit Shampoo gewaschen, nochmals gespült und anschließend getrocknet werden.

16. Färbemittel für Keratinfasern, insbesondere menschliche Keratinfasern wie dem Haar, **dadurch gekennzeichnet, daß** es die wie in den Ansprüchen 12 bis 15 definierten Komponenten (A) und (B) in getrennter Form umfaßt, wobei die Komponenten (A) und (B) entweder kurz vor der Verwendung gemischt oder nacheinander auf die zu behandelnden Fasern aufzutragen sind.

17. Vorrichtung mit mehreren Abteilen bzw. "FärbeKit", **dadurch gekennzeichnet, daß** es mindestens zwei Abteile umfaßt, von denen das eine die wie in Anspruch 12 definierte Komponente (A) beinhaltet und die zweiten die wie in Anspruch 12 oder 13 definierte Komponente (B) beinhaltet.

18. Einrichtung nach Anspruch 17, **dadurch gekennzeichnet, daß** die Komponente (A) und/oder die Komponente (B) in Form einer wasserfreien Zusammensetzung vorliegt bzw. vorliegen und daß die Vorrichtung ein drittes Abteil umfaßt, das ein zum Färben geeignetes kosmetisch annehmbares wäßriges Medium enthält, das vor Gebrauch in einem oder beiden ersten Abteil(en), das jeweils die Komponente (A) bzw. (B) umfaßt (umfassen), zu mischen ist.
